# EUROPEAN PATENT APPLICATION

(11) **EP 2 267 448 A1**
(43) Date of publication of application: **29.12.2010**
(21) Application number: 09729095.1
(22) Date of filing: 31.03.2009
(51) Int. Cl.: G01N 33/53, C07K 14/47, C07K 16/18, C12N 15/02, G01N 33/68

(54) **COMPOSITION, KIT AND METHOD FOR DETECTING NEUROPATHY**

(30) Priority: 31.03.2008 JP 2008091522
(71) Applicant: National Hospital Organization, Tokyo 152-8621 (JP); Santen Pharmaceutical Co., Ltd, Osaka-shi Osaka 533-8651 (JP)
(72) Inventor: IWATA, Takeshi, Tokyo 152-8902 (JP); MATSUNO, Kiyoshi, Osaka-shi Osaka 533-8651 (JP); TANAHASHI, Kazuhiro, Otsu-shi Shiga 520-8558 (JP)
(74) Representative: Sutcliffe, Nicholas Robert
(86) International application number: PCT/JP2009/056729
(87) International publication number: WO 2009/123225

(57) **Abstract**

The present invention relates to a method for detecting a disease accompanied with neuropathy such as glaucoma, comprising measuring and/or detecting one or more of polypeptides shown in SEQ ID NOS: 1 to 15, mutants thereof, or fragments thereof in a biological sample from a subject, and also to a composition or kit for diagnosis of a disease accompanied with neuropathy such as glaucoma.

SEQUENCE LISTING

<110>  National Hospital Organization
       Santen Pharmaceutical Co. Ltd.

<120>  Composition, kit and method for assaying neuropathy

<130>  NRS/FP6727234

<140>  09729095.1
<141>  2009-03-31

<150>  PCT/JP2009/056729
<151>  2009-03-31

<150>  JP 2008-091522
<151>  2008-03-31

<160>  15

<170>  PatentIn version 3.4

<210>  1
<211>  451
<212>  PRT
<213>  Homo sapiens

<400>  1

<210>  2
<211>  881
<212>  PRT
<213>  Homo sapiens

<400>  2

Met Phe Trp Lys Phe Asp Leu His Thr Ser Ser His Leu Asp Thr Leu
1               5                   10                  15

Leu Glu Arg Glu Asp Leu Ser Leu Pro Glu Leu Leu Asp Glu Glu Asp
            20                  25                  30

Val Leu Gln Glu Cys Lys Val Val Asn Arg Lys Leu Leu Asp Phe Leu
        35                  40                  45

Leu Gln Pro Pro His Leu Gln Ala Met Val Ala Trp Val Thr Gln Glu
    50                  55                  60

Pro Pro Asp Ser Gly Glu Glu Arg Leu Arg Tyr Lys Tyr Pro Ser Val
65                  70                  75                  80

Ala Cys Glu Ile Leu Thr Ser Asp Val Pro Gln Ile Asn Asp Ala Leu
                85                  90                  95

Gly Ala Asp Glu Ser Leu Leu Asn Arg Leu Tyr Gly Phe Leu Gln Ser
            100                 105                 110

Thr Gly Ser Leu Asn Pro Leu Leu Ala Ser Phe Phe Ser Lys Val Met
        115                 120                 125

Gly Ile Leu Ile Asn Arg Lys Thr Asp Gln Leu Val Ser Phe Leu Arg
    130                 135                 140

Lys Lys Asp Asp Phe Val Asp Leu Leu Leu Gln His Ile Gly Thr Ser
145                 150                 155                 160

Ala Ile Met Asp Leu Leu Leu Arg Leu Leu Thr Cys Val Glu Arg Pro
                165                 170                 175

Gln Leu Arg Gln Asp Val Val Asn Trp Leu Asn Glu Glu Lys Ile Val
            180                 185                 190

Gln Arg Leu Ile Glu Gln Ile His Pro Ser Lys Asp Glu Asn Gln His
        195                 200                 205

Ser Asn Ala Ser Gln Ser Leu Cys Asp Ile Ile Arg Leu Ser Arg Glu
    210                 215                 220

Gln Met Ile Gln Val Gln Asp Ser Pro Glu Pro Asp Gln Leu Leu Ala
225                 230                 235                 240

Thr Leu Glu Lys Gln Glu Thr Ile Glu Gln Leu Leu Ser Asn Met Phe
                245                 250                 255

Glu Gly Glu Gln Ser Gln Ser Val Ile Val Ser Gly Ile Gln Val Leu
            260                 265                 270

Leu Thr Leu Leu Glu Pro Arg Arg Pro Arg Ser Glu Ser Val Thr Val
        275                 280                 285

Asn Ser Phe Phe Ser Ser Val Asp Gly Gln Leu Glu Leu Leu Ala Gln
    290                 295                 300

Gly Ala Leu Glu Ser Thr Val Ser Ser Val Gly Ala Leu His Ala Leu
305                 310                 315                 320

Arg Pro Arg Leu Ser Cys Phe His Gln Leu Leu Leu Glu Pro Pro Lys
                325                 330                 335

Leu Glu Pro Leu Gln Met Thr Trp Gly Met Leu Ala Pro Pro Leu Gly
            340                 345                 350

Asn Thr Arg Leu His Val Val Lys Leu Leu Ala Ser Ala Leu Ser Ala
        355                 360                 365

Asn Asp Ala Ala Leu Thr His Glu Leu Leu Ala Leu Asp Val Pro Asn
    370                 375                 380

Thr Met Leu Asp Leu Phe Phe His Tyr Val Phe Asn Asn Phe Leu His
385                 390                 395                 400

Ala Gln Val Glu Gly Cys Val Ser Thr Met Leu Ser Leu Gly Pro Pro
                405                 410                 415

Pro Asp Ser Ser Pro Glu Thr Pro Ile Gln Asn Pro Val Val Lys His
            420                 425                 430

Leu Leu Gln Gln Cys Arg Leu Val Glu Arg Ile Leu Thr Ser Trp Glu
        435                 440                 445

Glu Asn Asp Arg Val Gln Cys Ala Gly Gly Pro Arg Lys Gly Tyr Met
    450                 455                 460

Gly His Leu Thr Arg Val Ala Gly Ala Leu Val Gln Asn Thr Glu Lys
465                 470                 475                 480

Gly Pro Asn Ala Glu Gln Leu Arg Gln Leu Leu Lys Glu Leu Pro Ser
                485                 490                 495

Glu Gln Gln Glu Gln Trp Glu Ala Phe Val Ser Gly Pro Leu Ala Glu
            500                 505                 510

Thr Asn Lys Lys Asn Met Val Asp Leu Val Asn Thr His His Leu His
        515                 520                 525

Ser Ser Ser Asp Asp Glu Asp Asp Arg Leu Lys Glu Phe Asn Phe Pro
    530                 535                 540

Glu Glu Ala Val Leu Gln Gln Ala Phe Met Asp Phe Gln Met Gln Arg
545                 550                 555                 560

Met Thr Ser Ala Phe Ile Asp His Phe Gly Phe Asn Asp Glu Glu Phe
                565                 570                 575

Gly Glu Gln Glu Glu Ser Val Asn Ala Pro Phe Asp Lys Thr Ala Asn
            580                 585                 590

Ile Thr Phe Ser Leu Asn Ala Asp Asp Glu Asn Pro Asn Ala Asn Leu
        595                 600                 605

Leu Glu Ile Cys Tyr Lys Asp Arg Ile Gln Gln Phe Asp Asp Asp Glu
    610                 615                 620

Glu Glu Glu Asp Glu Glu Glu Ala Gln Gly Ser Gly Glu Ser Asp Gly
625                 630                 635                 640

Glu Asp Gly Ala Trp Gln Gly Ser Gln Leu Ala Arg Gly Ala Arg Leu
                645                 650                 655

Gly Gln Pro Pro Gly Val Arg Ser Gly Gly Ser Thr Asp Ser Glu Asp
            660                 665                 670

Glu Glu Glu Glu Asp Glu Glu Glu Glu Glu Asp Glu Glu Gly Ile Gly
        675                 680                 685

Cys Ala Ala Arg Gly Gly Ala Thr Pro Leu Ser Tyr Pro Ser Pro Gly
    690                 695                 700

Pro Gln Pro Pro Gly Pro Ser Trp Thr Ala Thr Phe Asp Pro Val Pro
705                 710                 715                 720

Thr Asp Ala Pro Thr Ser Pro Arg Val Ser Gly Glu Glu Glu Leu His
                725                 730                 735

Thr Gly Pro Pro Ala Pro Gln Gly Pro Leu Ser Val Pro Gln Gly Leu
            740                 745                 750

Pro Thr Gln Ser Leu Ala Ser Pro Pro Ala Arg Asp Ala Leu Gln Leu
        755                 760                 765

Arg Ser Gln Asp Pro Thr Pro Pro Ser Ala Pro Gln Glu Ala Thr Glu
    770                 775                 780

Gly Ser Lys Val Thr Glu Pro Ser Ala Pro Cys Gln Ala Leu Val Ser
785                 790                 795                 800

Ile Gly Asp Leu Gln Ala Thr Phe His Gly Ile Arg Ser Ala Pro Ser
                805                 810                 815

Ser Ser Asp Ser Ala Thr Arg Asp Pro Ser Thr Ser Val Pro Ala Ser
            820                 825                 830

Gly Ala His Gln Pro Pro Gln Thr Thr Glu Gly Glu Lys Ser Pro Glu
        835                 840                 845

Pro Leu Gly Leu Pro Gln Ser Gln Ser Ala Gln Ala Leu Thr Pro Pro
    850                 855                 860

Pro Ile Pro Asn Gly Ser Ala Pro Glu Gly Pro Ala Ser Pro Gly Ser
865                 870                 875                 880

Gln

<210>  3
<211>  261
<212>  PRT
<213>  Homo sapiens

<400>  3

Met Asn Pro Asn Cys Ala Arg Cys Gly Lys Ile Val Tyr Pro Thr Glu
1               5                   10                  15

Lys Val Asn Cys Leu Asp Lys Phe Trp His Lys Ala Cys Phe His Cys
            20                  25                  30

Glu Thr Cys Lys Met Thr Leu Asn Met Lys Asn Tyr Lys Gly Tyr Glu
        35                  40                  45

Lys Lys Pro Tyr Cys Asn Ala His Tyr Pro Lys Gln Ser Phe Thr Met
    50                  55                  60

Val Ala Asp Thr Pro Glu Asn Leu Arg Leu Lys Gln Gln Ser Glu Leu
65                  70                  75                  80

Gln Ser Gln Val Arg Tyr Lys Glu Glu Phe Glu Lys Asn Lys Gly Lys
                85                  90                  95

Gly Phe Ser Val Val Ala Asp Thr Pro Glu Leu Gln Arg Ile Lys Lys

            100                 105                 110

Thr Gln Asp Gln Ile Ser Asn Ile Lys Tyr His Glu Glu Phe Glu Lys
        115                 120                 125

Ser Arg Met Gly Pro Ser Gly Gly Glu Gly Met Glu Pro Glu Arg Arg
    130                 135                 140

Asp Ser Gln Asp Gly Ser Ser Tyr Arg Arg Pro Leu Glu Gln Gln Gln
145                 150                 155                 160

Pro His His Ile Pro Thr Ser Ala Pro Val Tyr Gln Gln Pro Gln Gln
                165                 170                 175

Gln Pro Val Ala Gln Ser Tyr Gly Gly Tyr Lys Glu Pro Ala Ala Pro
            180                 185                 190

Val Ser Ile Gln Arg Ser Ala Pro Gly Gly Gly Gly Lys Arg Tyr Arg
        195                 200                 205

Ala Val Tyr Asp Tyr Ser Ala Ala Asp Glu Asp Glu Val Ser Phe Gln
    210                 215                 220

Asp Gly Asp Thr Ile Val Asn Val Gln Gln Ile Asp Asp Gly Trp Met
225                 230                 235                 240

Tyr Gly Thr Val Glu Arg Thr Gly Asp Thr Gly Met Leu Pro Ala Asn
                245                 250                 255

Tyr Val Glu Ala Ile
            260

<210> 4
<211> 211
<212> PRT
<213> Homo sapiens

<400> 4

Met Asp Asn Leu Ser Ser Glu Glu Ile Gln Gln Arg Ala His Gln Ile
1               5                   10                  15

Thr Asp Glu Ser Leu Glu Ser Thr Arg Arg Ile Leu Gly Leu Ala Ile
            20                  25                  30

Glu Ser Gln Asp Ala Gly Ile Lys Thr Ile Thr Met Leu Asp Glu Gln
        35                  40                  45

Lys Glu Gln Leu Asn Arg Ile Glu Glu Gly Leu Asp Gln Ile Asn Lys
    50                  55                  60

Asp Met Arg Glu Thr Glu Lys Thr Leu Thr Glu Leu Asn Lys Cys Cys
65                  70                  75                  80

Gly Leu Cys Val Cys Pro Cys Asn Arg Thr Lys Asn Phe Glu Ser Gly
                85                  90                  95

Lys Ala Tyr Lys Thr Thr Trp Gly Asp Gly Gly Glu Asn Ser Pro Cys
            100                 105                 110

Asn Val Val Ser Lys Gln Pro Gly Pro Val Thr Asn Gly Gln Leu Gln
        115                 120                 125

Gln Pro Thr Thr Gly Ala Ala Ser Gly Gly Tyr Ile Lys Arg Ile Thr
    130                 135                 140

Asn Asp Ala Arg Glu Asp Glu Met Glu Glu Asn Leu Thr Gln Val Gly
145                 150                 155                 160

Ser Ile Leu Gly Asn Leu Lys Asp Met Ala Leu Asn Ile Gly Asn Glu
                165                 170                 175

Ile Asp Ala Gln Asn Pro Gln Ile Lys Arg Ile Thr Asp Lys Ala Asp
            180                 185                 190

Thr Asn Arg Asp Arg Ile Asp Ile Ala Asn Ala Arg Ala Lys Lys Leu
        195                 200                 205

Ile Asp Ser
    210

<210>  5
<211>  1595
<212>  PRT
<213>  Homo sapiens

<400>  5

Met Ala Gly Ala Trp Leu Arg Trp Gly Leu Leu Leu Trp Ala Gly Leu
1               5                   10                  15

Leu Ala Ser Ser Ala His Gly Arg Leu Arg Arg Ile Thr Tyr Val Val
            20                  25                  30

His Pro Gly Pro Gly Leu Ala Ala Gly Ala Leu Pro Leu Ser Gly Pro
        35                  40                  45

Pro Ala Ser Ser Arg Thr Phe Asn Val Ala Leu Asn Ala Arg Tyr Ser
    50                  55                  60

Arg Ser Ser Ala Ala Ala Gly Ala Pro Ser Arg Ala Ser Pro Gly Val
65                  70                  75                  80

Pro Ser Glu Arg Thr Arg Arg Thr Ser Lys Pro Gly Gly Ala Ala Leu
                85                  90                  95

Gln Gly Leu Arg Pro Pro Pro Pro Pro Pro Pro Glu Pro Ala Arg Pro
            100                 105                 110

Ala Val Pro Gly Gly Gln Leu His Pro Asn Pro Gly Gly His Pro Ala
        115                 120                 125

Ala Ala Pro Phe Thr Lys Gln Gly Arg Gln Val Val Arg Ser Lys Val
    130                 135                 140

Pro Gln Glu Thr Gln Ser Gly Gly Gly Ser Arg Leu Gln Val His Gln
145                 150                 155                 160

Lys Gln Gln Leu Gln Gly Val Asn Val Cys Gly Gly Arg Cys Cys His
                165                 170                 175

Gly Trp Ser Lys Ala Pro Gly Ser Gln Arg Cys Thr Lys Pro Ser Cys
            180                 185                 190

Val Pro Pro Cys Gln Asn Gly Gly Met Cys Leu Arg Pro Gln Leu Cys
        195                 200                 205

Val Cys Lys Pro Gly Thr Lys Gly Lys Ala Cys Glu Thr Ile Ala Ala
    210                 215                 220

Gln Asp Thr Ser Ser Pro Val Phe Gly Gly Gln Ser Pro Gly Ala Ala
225                 230                 235                 240

Ser Ser Trp Gly Pro Pro Glu Gln Ala Ala Lys His Thr Ser Ser Lys
                245                 250                 255

Lys Ala Asp Thr Leu Pro Arg Val Ser Pro Val Ala Gln Met Thr Leu
            260                 265                 270

Thr Leu Lys Pro Lys Pro Ser Val Gly Leu Pro Gln Gln Ile His Ser
        275                 280                 285

Gln Val Thr Pro Leu Ser Ser Gln Ser Val Val Ile His His Gly Gln
    290                 295                 300

Thr Gln Glu Tyr Val Leu Lys Pro Lys Tyr Phe Pro Ala Gln Lys Gly
305                 310                 315                 320

Ile Ser Gly Glu Gln Ser Thr Glu Gly Ser Phe Pro Leu Arg Tyr Val
                325                 330                 335

Gln Asp Gln Val Ala Ala Pro Phe Gln Leu Gln Gly Val Lys Val Lys
            340                 345                 350

Phe Pro Pro Asn Ile Val Asn Ile His Val Lys His Pro Pro Glu Ala
        355                 360                 365

Ser Val Gln Ile His Gln Val Ser Arg Ile Asp Gly Pro Thr Gly Gln
    370                 375                 380

Lys Thr Lys Glu Ala Gln Pro Gly Gln Ser Gln Val Ser Tyr Gln Gly
385                 390                 395                 400

Leu Pro Val Gln Lys Thr Gln Thr Ile His Ser Thr Tyr Ser His Gln
                405                 410                 415

Gln Val Ile Pro His Val Tyr Pro Val Ala Ala Lys Thr Gln Leu Gly
            420                 425                 430

Arg Cys Phe Gln Glu Thr Ile Gly Ser Gln Cys Gly Lys Ala Leu Pro
        435                 440                 445

Gly Leu Ser Lys Gln Glu Asp Cys Cys Gly Thr Val Gly Thr Ser Trp
    450                 455                 460

Gly Phe Asn Lys Cys Gln Lys Cys Pro Lys Lys Pro Ser Tyr His Gly
465                 470                 475                 480

Tyr Asn Gln Met Met Glu Cys Leu Pro Gly Tyr Lys Arg Val Asn Asn
                485                 490                 495

Thr Phe Cys Gln Asp Ile Asn Glu Cys Gln Leu Gln Gly Val Cys Pro
            500                 505                 510

Asn Gly Glu Cys Leu Asn Thr Met Gly Ser Tyr Arg Cys Thr Cys Lys
        515                 520                 525

Ile Gly Phe Gly Pro Asp Pro Thr Phe Ser Ser Cys Val Pro Asp Pro
    530                 535                 540

Pro Val Ile Ser Glu Glu Lys Gly Pro Cys Tyr Arg Leu Val Ser Ser
545                 550                 555                 560

Gly Arg Gln Cys Met Tyr Pro Leu Ser Val His Leu Thr Lys Gln Leu
                565                 570                 575

Cys Cys Cys Ser Val Gly Lys Ala Gly Pro His Cys Glu Lys Cys Pro
            580                 585                 590

Leu Pro Gly Thr Ala Ala Phe Lys Glu Ile Cys Pro Gly Gly Met Gly
        595                 600                 605

Tyr Thr Val Ser Gly Val His Arg Arg Arg Pro Ile His His His Val
    610                 615                 620

Gly Lys Gly Pro Val Phe Val Lys Pro Lys Asn Thr Gln Pro Val Ala
625                 630                 635                 640

Lys Ser Thr His Pro Pro Pro Leu Pro Ala Lys Glu Glu Pro Val Glu
                645                 650                 655

Ala Leu Thr Phe Ser Arg Glu His Gly Ala Arg Ser Ala Glu Pro Glu
            660                 665                 670

Val Ala Thr Ala Pro Pro Glu Lys Glu Ile Pro Ser Leu Asp Gln Glu
        675                 680                 685

Lys Thr Lys Leu Glu Pro Gly Gln Pro Gln Leu Ser Pro Gly Ile Ser
    690                 695                 700

Ala Ile His Leu His Pro Gln Phe Pro Val Val Ile Glu Lys Thr Ser
705                 710                 715                 720

Pro Pro Val Pro Val Glu Val Ala Pro Glu Ala Ser Thr Ser Ser Ala
                725                 730                 735

Ser Gln Val Ile Ala Pro Thr Gln Val Thr Glu Ile Asn Glu Cys Thr
            740                 745                 750

Val Asn Pro Asp Ile Cys Gly Ala Gly His Cys Ile Asn Leu Pro Val
        755                 760                 765

Arg Tyr Thr Cys Ile Cys Tyr Glu Gly Tyr Arg Phe Ser Glu Gln Gln
    770                 775                 780

Arg Lys Cys Val Asp Ile Asp Glu Cys Thr Gln Val Gln His Leu Cys
785                 790                 795                 800

Ser Gln Gly Arg Cys Glu Asn Thr Glu Gly Ser Phe Leu Cys Ile Cys
                805                 810                 815

Pro Ala Gly Phe Met Ala Ser Glu Glu Gly Thr Asn Cys Ile Asp Val
            820                 825                 830

Asp Glu Cys Leu Arg Pro Asp Val Cys Gly Glu Gly His Cys Val Asn
        835                 840                 845

Thr Val Gly Ala Phe Arg Cys Glu Tyr Cys Asp Ser Gly Tyr Arg Met
    850                 855                 860

Thr Gln Arg Gly Arg Cys Glu Asp Ile Asp Glu Cys Leu Asn Pro Ser
865                 870                 875                 880

Thr Cys Pro Asp Glu Gln Cys Val Asn Ser Pro Gly Ser Tyr Gln Cys
                885                 890                 895

Val Pro Cys Thr Glu Gly Phe Arg Gly Trp Asn Gly Gln Cys Leu Asp
            900                 905                 910

Val Asp Glu Cys Leu Glu Pro Asn Val Cys Ala Asn Gly Asp Cys Ser
        915                 920                 925

Asn Leu Glu Gly Ser Tyr Met Cys Ser Cys His Lys Gly Tyr Thr Arg
    930                 935                 940

Thr Pro Asp His Lys His Cys Arg Asp Ile Asp Glu Cys Gln Gln Gly
945                 950                 955                 960

Asn Leu Cys Val Asn Gly Gln Cys Lys Asn Thr Glu Gly Ser Phe Arg
                965                 970                 975

Cys Thr Cys Gly Gln Gly Tyr Gln Leu Ser Ala Ala Lys Asp Gln Cys
            980                 985                 990

Glu Asp Ile Asp Glu Cys Gln His  Arg His Leu Cys Ala  His Gly Gln
        995                 1000                 1005

Cys Arg  Asn Thr Glu Gly Ser  Phe Gln Cys Val Cys  Asp Gln Gly
    1010                 1015                 1020

Tyr Arg  Ala Ser Gly Leu Gly  Asp His Cys Glu Asp  Ile Asn Glu
    1025                 1030                 1035

Cys Leu  Glu Asp Lys Ser Val  Cys Gln Arg Gly Asp  Cys Ile Asn
    1040                 1045                 1050

Thr Ala  Gly Ser Tyr Asp Cys  Thr Cys Pro Asp Gly  Phe Gln Leu
    1055                 1060                 1065

Asp Asp  Asn Lys Thr Cys Gln  Asp Ile Asn Glu Cys  Glu His Pro
    1070                 1075                 1080

Gly Leu  Cys Gly Pro Gln Gly  Glu Cys Leu Asn Thr  Glu Gly Ser
    1085                 1090                 1095

Phe His  Cys Val Cys Gln Gln  Gly Phe Ser Ile Ser  Ala Asp Gly
    1100                 1105                 1110

Arg Thr  Cys Glu Asp Ile Asp  Glu Cys Val Asn Asn  Thr Val Cys
    1115                 1120                 1125

Asp Ser  His Gly Phe Cys Asp  Asn Thr Ala Gly Ser  Phe Arg Cys
    1130                 1135                 1140

Leu Cys  Tyr Gln Gly Phe Gln  Ala Pro Gln Asp Gly  Gln Gly Cys
    1145                 1150                 1155

Val Asp  Val Asn Glu Cys Glu  Leu Leu Ser Gly Val  Cys Gly Glu
    1160                 1165                 1170

Ala Phe Cys Glu Asn Val Glu Gly Ser Phe Leu Cys Val Cys Ala
    1175                1180                1185

Asp Glu Asn Gln Glu Tyr Ser Pro Met Thr Gly Gln Cys Arg Ser
    1190                1195                1200

Arg Thr Ser Thr Asp Leu Asp Val Asp Val Asp Gln Pro Lys Glu
    1205                1210                1215

Glu Lys Lys Glu Cys Tyr Tyr Asn Leu Asn Asp Ala Ser Leu Cys
    1220                1225                1230

Asp Asn Val Leu Ala Pro Asn Val Thr Lys Gln Glu Cys Cys Cys
    1235                1240                1245

Thr Ser Gly Ala Gly Trp Gly Asp Asn Cys Glu Ile Phe Pro Cys
    1250                1255                1260

Pro Val Leu Gly Thr Ala Glu Phe Thr Glu Met Cys Pro Lys Gly
    1265                1270                1275

Lys Gly Phe Val Pro Ala Gly Glu Ser Ser Ser Glu Ala Gly Gly
    1280                1285                1290

Glu Asn Tyr Lys Asp Ala Asp Glu Cys Leu Leu Phe Gly Gln Glu
    1295                1300                1305

Ile Cys Lys Asn Gly Phe Cys Leu Asn Thr Arg Pro Gly Tyr Glu
    1310                1315                1320

Cys Tyr Cys Lys Gln Gly Thr Tyr Tyr Asp Pro Val Lys Leu Gln
    1325                1330                1335

Cys Phe Asp Met Asp Glu Cys Gln Asp Pro Ser Ser Cys Ile Asp
    1340                1345                1350

Gly Gln Cys Val Asn Thr Glu Gly Ser Tyr Asn Cys Phe Cys Thr
    1355                1360                1365

His Pro Met Val Leu Asp Ala Ser Glu Lys Arg Cys Ile Arg Pro
    1370                1375                1380

Ala Glu Ser Asn Glu Gln Ile Glu Glu Thr Asp Val Tyr Gln Asp
    1385                1390                1395

Leu Cys Trp Glu His Leu Ser Asp Glu Tyr Val Cys Ser Arg Pro
    1400                1405                1410

Leu Val Gly Lys Gln Thr Thr Tyr Thr Glu Cys Cys Cys Leu Tyr
    1415                1420                1425

Gly Glu Ala Trp Gly Met Gln Cys Ala Leu Cys Pro Leu Lys Asp
    1430                1435                1440

Ser Asp Asp Tyr Ala Gln Leu Cys Asn Ile Pro Val Thr Gly Arg
    1445                1450                1455

Arg Gln Pro Tyr Gly Arg Asp Ala Leu Val Asp Phe Ser Glu Gln
    1460                1465                1470

Tyr Thr Pro Glu Ala Asp Pro Tyr Phe Ile Gln Asp Arg Phe Leu
    1475                1480                1485

Asn Ser Phe Glu Glu Leu Gln Ala Glu Glu Cys Gly Ile Leu Asn
    1490                1495                1500

Gly Cys Glu Asn Gly Arg Cys Val Arg Val Gln Glu Gly Tyr Thr
    1505                1510                1515

Cys Asp Cys Leu Asp Gly Tyr His Leu Asp Thr Ala Lys Met Thr
    1520                1525                1530

Cys Phe Asp Val Asn Glu Cys Asp Glu Leu Asn Asn Arg Met Ser
    1535                1540                1545

Leu Cys Lys Asn Ala Lys Cys Ile Asn Thr Asp Gly Ser Tyr Lys
    1550                1555                1560

Cys Leu Cys Leu Pro Gly Tyr Val Pro Ser Asp Lys Pro Asn Tyr
    1565                1570                1575

Cys Thr Pro Leu Asn Thr Ala Leu Asn Leu Glu Lys Asp Ser Asp
    1580                1585                1590

Leu Glu
    1595

<210>  6
<211>  649
<212>  PRT
<213>  Homo sapiens

<400>  6

<210>  7
<211>  536
<212>  PRT
<213>  Homo sapiens

<400>  7

<210>  8
<211>  44
<212>  PRT
<213>  Homo sapiens

<400>  8

Met Ala Asp Lys Pro Asp Met Gly Glu Ile Ala Ser Phe Asp Lys Ala
1               5                   10                  15

Lys Leu Lys Lys Thr Glu Thr Gln Glu Lys Asn Thr Leu Pro Thr Lys
            20                  25                  30

Glu Thr Ile Glu Gln Glu Lys Arg Ser Glu Ile Ser
        35                  40

<210>  9
<211>  457
<212>  PRT
<213>  Homo sapiens

<400>  9
   Met Thr Thr Glu Asp Tyr Lys Lys Leu Ala Pro Tyr Asn Ile Arg Arg
   1               5                   10                  15
   Ser Ser Thr Ser Gly Asp Thr Glu Glu Glu Glu Glu Glu Glu Val Val
               20                  25                  30
   Pro Phe Ser Ser Asp Glu Gln Lys Arg Arg Ser Glu Ala Ala Ser Gly
           35                  40                  45
   Val Leu Arg Arg Thr Ala Pro Arg Glu His Ser Tyr Val Leu Ser Ala
       50                  55                  60
   Ala Lys Lys Ser Thr Gly Ser Pro Thr Gln Glu Thr Gln Ala Pro Phe
   65                  70                  75                  80
   Ile Ala Lys Arg Val Glu Val Val Glu Glu Asp Gly Pro Ser Glu Lys
                   85                  90                  95
   Ser Gln Asp Pro Pro Ala Leu Ala Arg Ser Thr Pro Gly Ser Asn Ser
               100                 105                 110
   Ser Arg Gly Glu Glu Ile Val Arg Leu Gln Ile Leu Thr Pro Arg Ala
           115                 120                 125
   Gly Leu Arg Leu Val Ala Pro Asp Val Glu Gly Met Ser Ser Ser Ala
       130                 135                 140
   Thr Ser Val Ser Ala Val Pro Ala Asp Arg Lys Ser Asn Ser Thr Ala
   145                 150                 155                 160
   Ala Gln Glu Asp Ala Lys Ala Asp Pro Lys Gly Ala Leu Ala Asp Tyr
                   165                 170                 175
   Glu Gly Lys Asp Val Ala Thr Arg Val Gly Glu Ala Trp Gln Glu Arg
               180                 185                 190
   Pro Gly Ala Pro Arg Gly Gly Gln Gly Asp Pro Ala Val Pro Ala Gln
           195                 200                 205
   Gln Pro Ala Asp Pro Ser Thr Pro Glu Arg Gln Ser Ser Pro Ser Gly
       210                 215                 220
   Ser Glu Gln Leu Val Arg Arg Glu Ser Cys Gly Ser Ser Val Leu Thr
   225                 230                 235                 240
   Asp Phe Glu Gly Lys Asp Val Ala Thr Lys Val Gly Glu Ala Trp Gln
                   245                 250                 255
   Asp Arg Pro Gly Ala Pro Arg Gly Gly Gln Gly Asp Pro Ala Val Pro
               260                 265                 270
   Thr Gln Gln Pro Ala Asp Pro Ser Thr Pro Glu Gln Gln Asn Ser Pro
           275                 280                 285
   Ser Gly Ser Glu Gln Phe Val Arg Arg Glu Ser Cys Thr Ser Arg Val
       290                 295                 300
   Arg Ser Pro Ser Ser Cys Met Val Thr Val Thr Val Thr Ala Thr Ser
   305                 310                 315                 320
   Glu Gln Pro His Ile Tyr Ile Pro Ala Pro Ala Ser Glu Leu Asp Ser
                   325                 330                 335
   Ser Ser Thr Thr Lys Gly Ile Leu Phe Val Lys Glu Tyr Val Asn Ala
               340                 345                 350
   Ser Glu Val Ser Ser Gly Lys Pro Val Ser Ala Arg Tyr Ser Asn Val
           355                 360                 365
   Ser Ser Ile Glu Asp Ser Phe Ala Met Glu Lys Lys Pro Pro Cys Gly
       370                 375                 380
   Ser Thr Pro Tyr Ser Glu Arg Thr Thr Gly Gly Ile Cys Thr Tyr Cys
   385                 390                 395                 400
   Asn Arg Glu Ile Arg Asp Cys Pro Lys Ile Thr Leu Glu His Leu Gly
                   405                 410                 415
   Ile Cys Cys His Glu Tyr Cys Phe Lys Cys Gly Ile Cys Ser Lys Pro
               420                 425                 430
   Met Gly Asp Leu Leu Asp Gln Ile Phe Ile His Arg Asp Thr Ile His
           435                 440                 445
   Cys Gly Lys Cys Tyr Glu Lys Leu Phe
       450                 455

<210>  10
<211>  736
<212>  PRT
<213>  Homo sapiens

<400>  10

<210>  11
<211>  1030
<212>  PRT
<213>  Homo sapiens

<400>  11

Met Lys Met Ala Asp Ala Lys Gln Lys Arg Asn Glu Gln Leu Lys Arg
1               5                   10                  15

Trp Ile Gly Ser Glu Thr Asp Leu Glu Pro Pro Val Val Lys Arg Gln
            20                  25                  30

Lys Thr Lys Val Lys Phe Asp Asp Gly Ala Val Phe Leu Ala Ala Cys
        35                  40                  45

Ser Ser Gly Asp Thr Asp Glu Val Leu Lys Leu Leu His Arg Gly Ala
    50                  55                  60

Asp Ile Asn Tyr Ala Asn Val Asp Gly Leu Thr Ala Leu His Gln Ala
65                  70                  75                  80

Cys Ile Asp Asp Asn Val Asp Met Val Lys Phe Leu Val Glu Asn Gly
                85                  90                  95

Ala Asn Ile Asn Gln Pro Asp Asn Glu Gly Trp Ile Pro Leu His Ala
            100                 105                 110

Ala Ala Ser Cys Gly Tyr Leu Asp Ile Ala Glu Phe Leu Ile Gly Gln
        115                 120                 125

Gly Ala His Val Gly Ala Val Asn Ser Glu Gly Asp Thr Pro Leu Asp
    130                 135                 140

Ile Ala Glu Glu Glu Ala Met Glu Glu Leu Leu Gln Asn Glu Val Asn
145                 150                 155                 160

Arg Gln Gly Val Asp Ile Glu Ala Ala Arg Lys Glu Glu Glu Arg Ile
                165                 170                 175

Met Leu Arg Asp Ala Arg Gln Trp Leu Asn Ser Gly His Ile Asn Asp

            180                 185                 190

Val Arg His Ala Lys Ser Gly Gly Thr Ala Leu His Val Ala Ala Ala
        195                 200                 205

Lys Gly Tyr Thr Glu Val Leu Lys Leu Leu Ile Gln Ala Gly Tyr Asp
    210                 215                 220

Val Asn Ile Lys Asp Tyr Asp Gly Trp Thr Pro Leu His Ala Ala Ala
225                 230                 235                 240

His Trp Gly Lys Glu Glu Ala Cys Arg Ile Leu Val Asp Asn Leu Cys
                245                 250                 255

Asp Met Glu Met Val Asn Lys Val Gly Gln Thr Ala Phe Asp Val Ala
            260                 265                 270

Asp Glu Asp Ile Leu Gly Tyr Leu Glu Glu Leu Gln Lys Lys Gln Asn
        275                 280                 285

Leu Leu His Ser Glu Lys Arg Asp Lys Lys Ser Pro Leu Ile Glu Ser
    290                 295                 300

Thr Ala Asn Met Asp Asn Asn Gln Ser Gln Lys Thr Phe Lys Asn Lys
305                 310                 315                 320

Glu Thr Leu Ile Ile Glu Pro Glu Lys Asn Ala Ser Arg Ile Glu Ser
                325                 330                 335

Leu Glu Gln Glu Lys Val Asp Glu Glu Glu Glu Gly Lys Lys Asp Glu
            340                 345                 350

Ser Ser Cys Ser Ser Glu Glu Asp Glu Glu Asp Asp Ser Glu Ser Glu
        355                 360                 365

Ala Glu Thr Asp Lys Thr Lys Pro Leu Ala Ser Val Thr Asn Ala Asn
    370                 375                 380

Thr Ser Ser Thr Gln Ala Ala Pro Val Ala Val Thr Thr Pro Thr Val
385                 390                 395                 400

Ser Ser Gly Gln Ala Thr Pro Thr Ser Pro Ile Lys Lys Phe Pro Thr
                405                 410                 415

Thr Ala Thr Lys Ile Ser Pro Lys Glu Glu Glu Arg Lys Asp Glu Ser
            420                 425                 430

Pro Ala Thr Trp Arg Leu Gly Leu Arg Lys Thr Gly Ser Tyr Gly Ala
        435                 440                 445

Leu Ala Glu Ile Thr Ala Ser Lys Glu Gly Gln Lys Glu Lys Asp Thr

450                 455                 460

Ala Gly Val Thr Arg Ser Ala Ser Ser Pro Arg Leu Ser Ser Ser Leu
465                 470                 475                 480

Asp Asn Lys Glu Lys Glu Lys Asp Ser Lys Gly Thr Arg Leu Ala Tyr
                485                 490                 495

Val Ala Pro Thr Ile Pro Arg Arg Leu Ala Ser Thr Ser Asp Ile Glu
            500                 505                 510

Glu Lys Glu Asn Arg Asp Ser Ser Ser Leu Arg Thr Ser Ser Ser Tyr
        515                 520                 525

Thr Arg Arg Lys Trp Glu Asp Asp Leu Lys Lys Asn Ser Ser Val Asn
    530                 535                 540

Glu Gly Ser Thr Tyr His Lys Ser Cys Ser Phe Gly Arg Arg Gln Asp
545                 550                 555                 560

Asp Leu Ile Ser Ser Ser Val Pro Ser Thr Thr Ser Thr Pro Thr Val
                565                 570                 575

Thr Ser Ala Ala Gly Leu Gln Lys Ser Leu Leu Ser Ser Thr Ser Thr
            580                 585                 590

Thr Thr Lys Ile Thr Thr Gly Ser Ser Ser Ala Gly Thr Gln Ser Ser
        595                 600                 605

Thr Ser Asn Arg Leu Trp Ala Glu Asp Ser Thr Glu Lys Glu Lys Asp
    610                 615                 620

Ser Val Pro Thr Ala Val Thr Ile Pro Val Ala Pro Thr Val Val Asn
625                 630                 635                 640

Ala Ala Ala Ser Thr Thr Thr Leu Thr Thr Thr Thr Ala Gly Thr Val
                645                 650                 655

Ser Ser Thr Thr Glu Val Arg Glu Arg Arg Arg Ser Tyr Leu Thr Pro
            660                 665                 670

Val Arg Asp Glu Glu Ser Glu Ser Gln Arg Lys Ala Arg Ser Arg Gln
        675                 680                 685

Ala Arg Gln Ser Arg Arg Ser Thr Gln Gly Val Thr Leu Thr Asp Leu
    690                 695                 700

Gln Glu Ala Glu Lys Thr Ile Gly Arg Ser Arg Ser Thr Arg Thr Arg
705                 710                 715                 720

Glu Gln Glu Asn Glu Glu Lys Glu Lys Glu Glu Lys Glu Lys Gln Asp

                725                 730                 735

Lys Glu Lys Gln Glu Glu Lys Lys Glu Ser Glu Thr Ser Arg Glu Asp
            740                 745                 750

Glu Tyr Lys Gln Lys Tyr Ser Arg Thr Tyr Asp Glu Thr Tyr Gln Arg
        755                 760                 765

Tyr Arg Pro Val Ser Thr Ser Ser Ser Thr Thr Pro Ser Ser Ser Leu
    770                 775                 780

Ser Thr Met Ser Ser Ser Leu Tyr Ala Ser Ser Gln Leu Asn Arg Pro
785                 790                 795                 800

Asn Ser Leu Val Gly Ile Thr Ser Ala Tyr Ser Arg Gly Ile Thr Lys
                805                 810                 815

Glu Asn Glu Arg Glu Gly Glu Lys Arg Glu Glu Glu Lys Glu Gly Glu
            820                 825                 830

Asp Lys Ser Gln Pro Lys Ser Ile Arg Glu Arg Arg Arg Pro Arg Glu
        835                 840                 845

Lys Arg Arg Ser Thr Gly Val Ser Phe Trp Thr Gln Asp Ser Asp Glu
    850                 855                 860

Asn Glu Gln Glu Gln Gln Ser Asp Thr Glu Glu Gly Ser Asn Lys Lys
865                 870                 875                 880

Glu Thr Gln Thr Asp Ser Ile Ser Arg Tyr Glu Thr Ser Ser Thr Ser
                885                 890                 895

Ala Gly Asp Arg Tyr Asp Ser Leu Leu Gly Arg Ser Gly Ser Tyr Ser
            900                 905                 910

Tyr Leu Glu Glu Arg Lys Pro Tyr Ser Ser Arg Leu Glu Lys Asp Asp
        915                 920                 925

Ser Thr Asp Phe Lys Lys Leu Tyr Glu Gln Ile Leu Ala Glu Asn Glu
    930                 935                 940

Lys Leu Lys Ala Gln Leu His Asp Thr Asn Met Glu Leu Thr Asp Leu
945                 950                 955                 960

Lys Leu Gln Leu Glu Lys Ala Thr Gln Arg Gln Glu Arg Phe Ala Asp
                965                 970                 975

Arg Ser Leu Leu Glu Met Glu Lys Arg Glu Arg Arg Ala Leu Glu Arg
            980                 985                 990

Arg Ile Ser Glu Met Glu Glu Glu Leu Lys Met Leu Pro Asp Leu Lys

995                 1000                1005

Ala Asp  Asn Gln Arg Leu Lys  Asp Glu Asn Gly Ala  Leu Ile Arg
    1010                 1015                 1020

Val Ile  Ser Lys Leu Ser Lys
    1025                 1030

<210>  12
<211>  738
<212>  PRT
<213>  Homo sapiens

<400>  12

Met Gln Pro Arg Trp Ala Gln Gly Ala Thr Met Trp Leu Gly Val Leu
1               5                   10                  15

Leu Thr Leu Leu Leu Cys Ser Ser Leu Glu Gly Gln Glu Asn Ser Phe
            20                  25                  30

Thr Ile Asn Ser Val Asp Met Lys Ser Leu Pro Asp Trp Thr Val Gln
        35                  40                  45

Asn Gly Lys Asn Leu Thr Leu Gln Cys Phe Ala Asp Val Ser Thr Thr
    50                  55                  60

Ser His Val Lys Pro Gln His Gln Met Leu Phe Tyr Lys Asp Asp Val
65                  70                  75                  80

Leu Phe Tyr Asn Ile Ser Ser Met Lys Ser Thr Glu Ser Tyr Phe Ile
                85                  90                  95

Pro Glu Val Arg Ile Tyr Asp Ser Gly Thr Tyr Lys Cys Thr Val Ile
            100                 105                 110

Val Asn Asn Lys Glu Lys Thr Thr Ala Glu Tyr Gln Leu Leu Val Glu
        115                 120                 125

Gly Val Pro Ser Pro Arg Val Thr Leu Asp Lys Lys Glu Ala Ile Gln
    130                 135                 140

Gly Gly Ile Val Arg Val Asn Cys Ser Val Pro Glu Glu Lys Ala Pro
145                 150                 155                 160

Ile His Phe Thr Ile Glu Lys Leu Glu Leu Asn Glu Lys Met Val Lys
                165                 170                 175

Leu Lys Arg Glu Lys Asn Ser Arg Asp Gln Asn Phe Val Ile Leu Glu
            180                 185                 190

Phe Pro Val Glu Glu Gln Asp Arg Val Leu Ser Phe Arg Cys Gln Ala
        195                 200                 205

Arg Ile Ile Ser Gly Ile His Met Gln Thr Ser Glu Ser Thr Lys Ser
    210                 215                 220

Glu Leu Val Thr Val Thr Glu Ser Phe Ser Thr Pro Lys Phe His Ile
225                 230                 235                 240

Ser Pro Thr Gly Met Ile Met Glu Gly Ala Gln Leu His Ile Lys Cys
                245                 250                 255

Thr Ile Gln Val Thr His Leu Ala Gln Glu Phe Pro Glu Ile Ile Ile
            260                 265                 270

Gln Lys Asp Lys Ala Ile Val Ala His Asn Arg His Gly Asn Lys Ala
        275                 280                 285

Val Tyr Ser Val Met Ala Met Val Glu His Ser Gly Asn Tyr Thr Cys
    290                 295                 300

Lys Val Glu Ser Ser Arg Ile Ser Lys Val Ser Ser Ile Val Val Asn
305                 310                 315                 320

Ile Thr Glu Leu Phe Ser Lys Pro Glu Leu Glu Ser Ser Phe Thr His
                325                 330                 335

Leu Asp Gln Gly Glu Arg Leu Asn Leu Ser Cys Ser Ile Pro Gly Ala
            340                 345                 350

Pro Pro Ala Asn Phe Thr Ile Gln Lys Glu Asp Thr Ile Val Ser Gln
        355                 360                 365

Thr Gln Asp Phe Thr Lys Ile Ala Ser Lys Ser Asp Ser Gly Thr Tyr
    370                 375                 380

Ile Cys Thr Ala Gly Ile Asp Lys Val Val Lys Lys Ser Asn Thr Val
385                 390                 395                 400

Gln Ile Val Val Cys Glu Met Leu Ser Gln Pro Arg Ile Ser Tyr Asp
                405                 410                 415

Ala Gln Phe Glu Val Ile Lys Gly Gln Thr Ile Glu Val Arg Cys Glu
            420                 425                 430

Ser Ile Ser Gly Thr Leu Pro Ile Ser Tyr Gln Leu Leu Lys Thr Ser
        435                 440                 445

Lys Val Leu Glu Asn Ser Thr Lys Asn Ser Asn Asp Pro Ala Val Phe
    450                 455                 460

Lys Asp Asn Pro Thr Glu Asp Val Glu Tyr Gln Cys Val Ala Asp Asn
465                 470                 475                 480

Cys His Ser His Ala Lys Met Leu Ser Glu Val Leu Arg Val Lys Val
                485                 490                 495

Ile Ala Pro Val Asp Glu Val Gln Ile Ser Ile Leu Ser Ser Lys Val
            500                 505                 510

Val Glu Ser Gly Glu Asp Ile Val Leu Gln Cys Ala Val Asn Glu Gly
        515                 520                 525

Ser Gly Pro Ile Thr Tyr Lys Phe Tyr Arg Glu Lys Glu Gly Lys Pro
    530                 535                 540

Phe Tyr Gln Met Thr Ser Asn Ala Thr Gln Ala Phe Trp Thr Lys Gln
545                 550                 555                 560

Lys Ala Ser Lys Glu Gln Glu Gly Glu Tyr Tyr Cys Thr Ala Phe Asn
                565                 570                 575

Arg Ala Asn His Ala Ser Ser Val Pro Arg Ser Lys Ile Leu Thr Val
            580                 585                 590

Arg Val Ile Leu Ala Pro Trp Lys Lys Gly Leu Ile Ala Val Val Ile
        595                 600                 605

Ile Gly Val Ile Ile Ala Leu Leu Ile Ile Ala Ala Lys Cys Tyr Phe
    610                 615                 620

Leu Arg Lys Ala Lys Ala Lys Gln Met Pro Val Glu Met Ser Arg Pro
625                 630                 635                 640

Ala Val Pro Leu Leu Asn Ser Asn Asn Glu Lys Met Ser Asp Pro Asn
                645                 650                 655

Met Glu Ala Asn Ser His Tyr Gly His Asn Asp Asp Val Arg Asn His
            660                 665                 670

Ala Met Lys Pro Ile Asn Asp Asn Lys Glu Pro Leu Asn Ser Asp Val
        675                 680                 685

Gln Tyr Thr Glu Val Gln Val Ser Ser Ala Glu Ser His Lys Asp Leu
    690                 695                 700

Gly Lys Lys Asp Thr Glu Thr Val Tyr Ser Glu Val Arg Lys Ala Val
705                 710                 715                 720

Pro Asp Ala Val Glu Ser Arg Tyr Ser Arg Thr Glu Gly Ser Leu Asp
                725                 730                 735

Gly Thr

<210>  13
<211>  199
<212>  PRT
<213>  Homo sapiens

<400>  13

Met Ala Asn Arg Gly Pro Ala Tyr Gly Leu Ser Arg Glu Val Gln Gln
1               5                   10                  15

Lys Ile Glu Lys Gln Tyr Asp Ala Asp Leu Glu Gln Ile Leu Ile Gln
            20                  25                  30

Trp Ile Thr Thr Gln Cys Arg Lys Asp Val Gly Arg Pro Gln Pro Gly
        35                  40                  45

Arg Glu Asn Phe Gln Asn Trp Leu Lys Asp Gly Thr Val Leu Cys Glu
    50                  55                  60

Leu Ile Asn Ala Leu Tyr Pro Glu Gly Gln Ala Pro Val Lys Lys Ile
65                  70                  75                  80

Gln Ala Ser Thr Met Ala Phe Lys Gln Met Glu Gln Ile Ser Gln Phe
                85                  90                  95

Leu Gln Ala Ala Glu Arg Tyr Gly Ile Asn Thr Thr Asp Ile Phe Gln
            100                 105                 110

Thr Val Asp Leu Trp Glu Gly Lys Asn Met Ala Cys Val Gln Arg Thr
        115                 120                 125

Leu Met Asn Leu Gly Gly Leu Ala Val Ala Arg Asp Asp Gly Leu Phe
    130                 135                 140

Ser Gly Asp Pro Asn Trp Phe Pro Lys Lys Ser Lys Glu Asn Pro Arg
145                 150                 155                 160

Asn Phe Ser Asp Asn Gln Leu Gln Glu Gly Lys Asn Val Ile Gly Leu
                165                 170                 175

Gln Met Gly Thr Asn Arg Gly Ala Ser Gln Ala Gly Met Thr Gly Tyr
            180                 185                 190

Gly Met Pro Arg Gln Ile Leu
        195


<210>  14
<211>  142
<212>  PRT
<213>  Homo sapiens

<400>  14

Met Ile Arg Phe Ile Leu Ile Gln Asn Arg Ala Gly Lys Thr Arg Leu
1               5                   10                  15

Ala Lys Trp Tyr Met Gln Phe Asp Asp Asp Glu Lys Gln Lys Leu Ile
            20                  25                  30

Glu Glu Val His Ala Val Val Thr Val Arg Asp Ala Lys His Thr Asn
        35                  40                  45

Phe Val Glu Phe Arg Asn Phe Lys Ile Ile Tyr Arg Arg Tyr Ala Gly
    50                  55                  60

Leu Tyr Phe Cys Ile Cys Val Asp Val Asn Asp Asn Asn Leu Ala Tyr
65                  70                  75                  80

Leu Glu Ala Ile His Asn Phe Val Glu Val Leu Asn Glu Tyr Phe His
                85                  90                  95

Asn Val Cys Glu Leu Asp Leu Val Phe Asn Phe Tyr Lys Val Tyr Thr
            100                 105                 110

Val Val Asp Glu Met Phe Leu Ala Gly Glu Ile Arg Glu Thr Ser Gln
        115                 120                 125

Thr Lys Val Leu Lys Gln Leu Leu Met Leu Gln Ser Leu Glu
    130                 135                 140

<210>  15
<211>  1087
<212>  PRT
<213>  Homo sapiens

<400>  15

Met Ala Asp His Val Gln Ser Leu Ala Gln Leu Glu Asn Leu Cys Lys
1               5                   10                  15

Gln Leu Tyr Glu Thr Thr Asp Thr Thr Thr Arg Leu Gln Ala Glu Lys
            20                  25                  30

Ala Leu Val Glu Phe Thr Asn Ser Pro Asp Cys Leu Ser Lys Cys Gln
        35                  40                  45

Leu Leu Leu Glu Arg Gly Ser Ser Ser Tyr Ser Gln Leu Leu Ala Ala
    50                  55                  60

Thr Cys Leu Thr Lys Leu Val Ser Arg Thr Asn Asn Pro Leu Pro Leu
65                  70                  75                  80

Glu Gln Arg Ile Asp Ile Arg Asn Tyr Val Leu Asn Tyr Leu Ala Thr
                85                  90                  95

Arg Pro Lys Leu Ala Thr Phe Val Thr Gln Ala Leu Ile Gln Leu Tyr

            100                 105                 110

Ala Arg Ile Thr Lys Leu Gly Trp Phe Asp Cys Gln Lys Asp Asp Tyr
        115                 120                 125

Val Phe Arg Asn Ala Ile Thr Asp Val Thr Arg Phe Leu Gln Asp Ser
    130                 135                 140

Val Glu Tyr Cys Ile Ile Gly Val Thr Ile Leu Ser Gln Leu Thr Asn
145                 150                 155                 160

Glu Ile Asn Gln Ala Asp Thr Thr His Pro Leu Thr Lys His Arg Lys
                165                 170                 175

Ile Ala Ser Ser Phe Arg Asp Ser Ser Leu Phe Asp Ile Phe Thr Leu
            180                 185                 190

Ser Cys Asn Leu Leu Lys Gln Ala Ser Gly Lys Asn Leu Asn Leu Asn
        195                 200                 205

Asp Glu Ser Gln His Gly Leu Leu Met Gln Leu Leu Lys Leu Thr His
    210                 215                 220

Asn Cys Leu Asn Phe Asp Phe Ile Gly Thr Ser Thr Asp Glu Ser Ser
225                 230                 235                 240

Asp Asp Leu Cys Thr Val Gln Ile Pro Thr Ser Trp Arg Ser Ala Phe
                245                 250                 255

Leu Asp Ser Ser Thr Leu Gln Leu Phe Phe Asp Leu Tyr His Ser Ile
            260                 265                 270

Pro Pro Ser Phe Ser Pro Leu Val Leu Ser Cys Leu Val Gln Ile Ala
        275                 280                 285

Ser Val Arg Arg Ser Leu Phe Asn Asn Ala Glu Arg Ala Lys Phe Leu
    290                 295                 300

Ser His Leu Val Asp Gly Val Lys Arg Ile Leu Glu Asn Pro Gln Ser
305                 310                 315                 320

Leu Ser Asp Pro Asn Asn Tyr His Glu Phe Cys Arg Leu Leu Ala Arg
                325                 330                 335

Leu Lys Ser Asn Tyr Gln Leu Gly Glu Leu Val Lys Val Glu Asn Tyr
            340                 345                 350

Pro Glu Val Ile Arg Leu Ile Ala Asn Phe Thr Val Thr Ser Leu Gln
        355                 360                 365

His Trp Glu Phe Ala Pro Asn Ser Val His Tyr Leu Leu Ser Leu Trp

370                 375                 380

Gln Arg Leu Ala Ala Ser Val Pro Tyr Val Lys Ala Thr Glu Pro His
385                 390                 395                 400

Met Leu Glu Thr Tyr Thr Pro Glu Val Thr Lys Ala Tyr Ile Thr Ser
                405                 410                 415

Arg Leu Glu Ser Val His Ile Ile Leu Arg Asp Gly Leu Glu Asp Pro
            420                 425                 430

Leu Glu Asp Thr Gly Leu Val Gln Gln Gln Leu Asp Gln Leu Ser Thr
        435                 440                 445

Ile Gly Arg Cys Glu Tyr Glu Lys Thr Cys Ala Leu Leu Val Gln Leu
    450                 455                 460

Phe Asp Gln Ser Ala Gln Ser Tyr Gln Glu Leu Leu Gln Ser Ala Ser
465                 470                 475                 480

Ala Ser Pro Met Asp Ile Ala Val Gln Glu Gly Arg Leu Thr Trp Leu
                485                 490                 495

Val Tyr Ile Ile Gly Ala Val Ile Gly Gly Arg Val Ser Phe Ala Ser
            500                 505                 510

Thr Asp Glu Gln Asp Ala Met Asp Gly Glu Leu Val Cys Arg Val Leu
        515                 520                 525

Gln Leu Met Asn Leu Thr Asp Ser Arg Leu Ala Gln Ala Gly Asn Glu
    530                 535                 540

Lys Leu Glu Leu Ala Met Leu Ser Phe Phe Glu Gln Phe Arg Lys Ile
545                 550                 555                 560

Tyr Ile Gly Asp Gln Val Gln Lys Ser Ser Lys Leu Tyr Arg Arg Leu
                565                 570                 575

Ser Glu Val Leu Gly Leu Asn Asp Glu Thr Met Val Leu Ser Val Phe
            580                 585                 590

Ile Gly Lys Ile Ile Thr Asn Leu Lys Tyr Trp Gly Arg Cys Glu Pro
        595                 600                 605

Ile Thr Ser Lys Thr Leu Gln Leu Leu Asn Asp Leu Ser Ile Gly Tyr
    610                 615                 620

Ser Ser Val Arg Lys Leu Val Lys Leu Ser Ala Val Gln Phe Met Leu
625                 630                 635                 640

Asn Asn His Thr Ser Glu His Phe Ser Phe Leu Gly Ile Asn Asn Gln

645                 650                 655

Ser Asn Leu Thr Asp Met Arg Cys Arg Thr Thr Phe Tyr Thr Ala Leu
            660                 665                 670

Gly Arg Leu Leu Met Val Asp Leu Gly Glu Asp Glu Asp Gln Tyr Glu
        675                 680                 685

Gln Phe Met Leu Pro Leu Thr Ala Ala Phe Glu Ala Val Ala Gln Met
    690                 695                 700

Phe Ser Thr Asn Ser Phe Asn Glu Gln Glu Ala Lys Arg Thr Leu Val
705                 710                 715                 720

Gly Leu Val Arg Asp Leu Arg Gly Ile Ala Phe Ala Phe Asn Ala Lys
                725                 730                 735

Thr Ser Phe Met Met Leu Phe Glu Trp Ile Tyr Pro Ser Tyr Met Pro
            740                 745                 750

Ile Leu Gln Arg Ala Ile Glu Leu Trp Tyr His Asp Pro Ala Cys Thr
        755                 760                 765

Thr Pro Val Leu Lys Leu Met Ala Glu Leu Val His Asn Arg Ser Gln
    770                 775                 780

Arg Leu Gln Phe Asp Val Ser Ser Pro Asn Gly Ile Leu Leu Phe Arg
785                 790                 795                 800

Glu Thr Ser Lys Met Ile Thr Met Tyr Gly Asn Arg Ile Leu Thr Leu
                805                 810                 815

Gly Glu Val Pro Lys Asp Gln Val Tyr Ala Leu Lys Leu Lys Gly Ile
            820                 825                 830

Ser Ile Cys Phe Ser Met Leu Lys Ala Ala Leu Ser Gly Ser Tyr Val
        835                 840                 845

Asn Phe Gly Val Phe Arg Leu Tyr Gly Asp Asp Ala Leu Asp Asn Ala
    850                 855                 860

Leu Gln Thr Phe Ile Lys Leu Leu Leu Ser Ile Pro His Ser Asp Leu
865                 870                 875                 880

Leu Asp Tyr Pro Lys Leu Ser Gln Ser Tyr Tyr Ser Leu Leu Glu Val
                885                 890                 895

Leu Thr Gln Asp His Met Asn Phe Ile Ala Ser Leu Glu Pro His Val
            900                 905                 910

Ile Met Tyr Ile Leu Ser Ser Ile Ser Glu Gly Leu Thr Ala Leu Asp

        915                 920                 925

Thr Met Val Cys Thr Gly Cys Cys Ser Cys Leu Asp His Ile Val Thr
    930                 935                 940

Tyr Leu Phe Lys Gln Leu Ser Arg Ser Thr Lys Lys Arg Thr Thr Pro
945                 950                 955                 960

Leu Asn Gln Glu Ser Asp Arg Phe Leu His Ile Met Gln Gln His Pro
                965                 970                 975

Glu Met Ile Gln Gln Met Leu Ser Thr Val Leu Asn Ile Ile Ile Phe
            980                 985                 990

Glu Asp Cys Arg Asn Gln Trp Ser Met Ser Arg Pro Leu Leu Gly Leu
        995                 1000                1005

Ile Leu Leu Asn Glu Lys Tyr Phe Ser Asp Leu Arg Asn Ser Ile
    1010                1015                1020

Val Asn Ser Gln Pro Pro Glu Lys Gln Gln Ala Met His Leu Cys
    1025                1030                1035

Phe Glu Asn Leu Met Glu Gly Ile Glu Arg Asn Leu Leu Thr Lys
    1040                1045                1050

Asn Arg Asp Arg Phe Thr Gln Asn Leu Ser Ala Phe Arg Arg Glu
    1055                1060                1065

Val Asn Asp Ser Met Lys Asn Ser Thr Tyr Gly Val Asn Ser Asn
    1070                1075                1080

Asp Met Met Ser
    1085

## Description

### TECHNICAL FIELD

The present invention relates to a composition or a kit useful for diagnosis of neuropathy.

The present invention also relates to a method for assaying (or determining or identifying) neuropathy using the composition or the kit.

### BACKGROUND ART

Due to progress of medical technology and changes in social environments, diseases that develop or progress in association with ageing have been highlited in recent years. As represented by lifestyle-associated diseases, it is said that such diseases develop and progress as a result of the gradual accumulation of small changes occurring in the living body. In particular, diseases caused mainly by neuropathy have become serious social problems.

The term "neuropathy" (or a neurological disorder) refers to a condition in which stenosis and/or occlusion of peripheral blood vessels that supply oxygen and nutrients to tissues is caused by arteriosclerosis or the like, resulting in stagnation of blood flow and insufficient supply of nutrients to peripharal tissues, and eventually leading to an abnormal state of nerve functions. The development of a vascular disorder causes serious problems such as neuropathy in an organ or tissue which is rich in vasculature. If a vascular disorder develops in a sensory organ, and particularly in ocular tissue, it might result in blindness.

A fluid called "aqueous humor" flows in the eyes and serves in place of blood so as to deliver nutrition and the like. Aqueous humor is produced in the ciliary body and is discharged from the Schlemm's canal. The eye shape is maintained by the aqueous humor pressure which refers to "intraocular pressure." Intraocular pressure slightly varies depending on season or time of day, but it is maintained at an almost constant level.

Glaucoma is a disease asociated with visual field constriction and optic nerve disorder caused by a certain cause. An increase in intraocular pressure is said to be a pathological cause of the disease. The disease causes blindness of the elderly. Along with a sharp increase in the elderly population in recent years, the number of glaucoma patients has been continously increasing.

Glaucoma is asymptomatic and thus it is difficult to detect glaucoma at an early stage. Since glaucoma can cause blindness, it is very important to diagnose the disease at an early stage. Hitherto, for the diagnosis of glaucoma, funduscopy has mainly been performed. Prior to the examination, a mydriatic agent that allows the pupil to dilate is administered to a patient, and subsequently, a physician directly observes the retina with a funduscope or fundus camera. However, when the pupils are allowed to dilate under the influence of a mydriatic agent, the flow of aqueous humor becomes stagnant, resulting in increase of intraocular pressure. Therefore, at present, it cannot be said that the use of such agent will never cause further deterioration of pathological conditions. Moreover, it cannot always be said that direct observation of the retina by a physician is an objective assay method. In direct observation, every patient must be examined by a phisican, and thus it is difficult to apply such examination to mass-screening for the examination of many subjects.

As described above, there are rather many problems in relation to existing examination methods. Hence, a high-throughput assay method for early diagnosis that is less stressful for patients has been awaited, whereby the degree of pathological progression and time-dependent changes during treatment can be objectively and quantitatively determined for patients.

An assay method using a diagnosis marker is an objective high-throughput method. In the past, a method for diagnosing glaucoma using an antibody that specifically recognizes TIGR protein, which is a glucocorticoid-induced protein produced by trabecular meshwork cells, (Japanese Patent Publication (Kohyo) No. 10-509866 A (1998)), as well as quantification of TGF-β in aqueous humor (Min SH, Lee TI, Chung YS, Kim HK., Transforming growth factor-beta levels in human aqueous humor of glaucomatous, diabetic and uveitic eyes. Korean J Ophthalmol. 2006 Sep;20(3):162-5.), have been disclosed. In these methods, glaucoma cannot be determined with relatively high specificity with the use of such markers, and the ocular tissue that is not easy to take for diagnosis purpose is used as a specimen. Therefore, the methods are still research-stage methods under the present circumstances.

Along with the recent progress in genome analysis (genomics) and proteome analysis (proteomics), a variety of novel marker candidates have been reported. For glaucoma, as a result of proteome analysis using an ocular tissue, a variety of novel marker candidates have been reported (Bhuattacharya SK, Crabb JS, Bonilha VL, Gu X, Takahara H, Crabb JW., Proteomics implicates peptidyl arginine deiminase 2 and optic nerve citrullination in glaucoma pathogenesis., Invest Ophthalmol Vis Sci. 2006 Jun;47(6):2508-14., and Tezel G, Tang X, Cai J., Proteomic identification of oxidatively modified retinal proteins in a chronic pressure-induced rat model of glaucoma, Invest Ophthalmol Vis Sci. 2005 Sep; 46(9):3177-3187). However, there is no report on protein markers for glaucoma found by proteome analysis using blood specimens. Also, there is no known method for diagnosing the glaucoma using protein markers in bloods from glaucoma patients. It is expected that if markers allowing diagnosis of glaucoma and diagnosis methods using such markers can be created, such markers or methods will be widely used for diagnosis of neuropathy itself.

### DISCLOSURE OF THE INVENTION

### PROBLEM TO BE SOLVED BY THE INVENTION

However, the above-mentioned known markers and marker candidates have poor specificity and/or sensitivity, and efficient methods for detecting such markers from biological samples have not been established. Because, in general, these markers are not clinically used, there are high demands on markers with higher specificity and sensitivity for neuropathy. In addition, a high-throughput assay method that is less stressful for patients has been awaited, whereby degrees of pathological progression, as well as post-surgical time-dependent changes, can be objectively and quantitatively determined for patients.

An object of the present invention is to provide a composition or kit useful for diagnosis of a disease accompanied with neuropathy, particularly glaucoma, and a method for assaying a disease accompanied with neuropathy using the composition or kit.

### MEANS FOR SOLVING THE PROBLEM

The present inventors have now found blood protein markers specifically detected in glaucoma patients by subjecting blood specimens of patients with glaucoma and blood specimens of patients with another ocular diseases to proteome analysis. This finding led to the complesion of an invention drawn to a method for determining glaucoma using said protein markers.

### <Summary of the Invention>

The present invention has the following characteristics.

(1) A method for determining neuropathy, comprising quantitatively or qualitatively measuring and/or detecting one or more of polypeptides comprising any of amino acid sequences shown in SEQ ID NOS: 1 to 15, mutants thereof, or fragments thereof in a biological sample from a subject.

(2) The method according to (1), wherein the neuropathy is ocular tissue neuropathy.

(3) The method according to (2), wherein the ocular tissue neuropathy is glaucoma.

(4) The method according to any one of (1) to (3), wherein the measurement and/or detection of the polypeptide, a mutant thereof, or a fragment thereof is carried out by mass spectrometry.

(5) The method according to (4), wherein the measurement and/or detection is carried out using a substance capable of binding to the polypeptide, a mutant thereof, or a fragment thereof.

(6) The method according to (5), wherein the substance capable of binding is an antibody or an antigen-binding fragment thereof.

(7) The method according to (6), wherein the antibody labeled with any of an enzyme, a fluorophor, a dye, a radioisotope, or biotin is used.

(8) The method according to (6) or (7), wherein the antibody or an antigen-binding fragment thereof is a monoclonal antibody or a polyclonal antibody, or an antigen-binding fragment thereof.

(9) The method according to any one of (1) to (8), wherein the biological sample is blood, plasma, or serum.

(10) A composition for diagnosis and/or detection of neuropathy, which comprises one or more antibody probes selected from antibodies, antigen-binding fragments, or chemically modified derivatives thereof capable of specifically binding to at least one of polypeptides comprising any of amino acid sequences shown in SEQ ID NOS: 1 to 15, mutants thereof, or fragments thereof.

(11) A kit for diagnosis and/or detection of neuropathy, which comprises one or more antibody probes selected from antibodies, antigen-binding fragments, or chemically modified derivatives thereof capable of specifically binding to at least one of polypeptides comprising any of amino acid sequences shown in SEQ ID NOS: 1 to 15, mutants thereof, or fragments thereof.

(12) The composition or kit for diagnosis and/or detection according to (10) or (11), wherein the neuropathy is ocular tissue neuropathy.

(13) The composition or kit for diagnosis and/or detection according to (12), wherein the ocular tissue neuropathy is glaucoma.

(14) Use of one or more antibody probes selected from antibodies, antigen-binding fragments, or chemically modified derivatives thereof capable of specifically binding to at least one of polypeptide comprising any of amino acid sequences shown in SEQ ID NOS: 1 to 15, mutants thereof, or fragments thereof, in production of the kit according to any one of (11)-(13).

### <Definition>

Terms as used herein comprise definitions as described below.

Herein, mutants of polypeptides comprising any of amino acid sequences shown in SEQ ID NOS: 1 to 15 correspond to mutants comprising a deletion(s), substitution(s), addition(s), or insertion(s) of one or more, preferably one or several, amino acids in the amino acid sequences shown in SEQ ID NOS: 1-15 or partial sequences thereof; or mutants comprising amino acid sequences showing about 80% or more, about 85% or more, preferably about 90% or more, more preferably about 95% or more, about 97% or more, about 98% or more, or about 99% or more identity with the amino acid sequences or partial sequences thereof.

The term "% identity" as used herein generally refers to the percentage (%) of the number of amino acid residues or positions that are identical in two amino acid seqeunces relative to the total number of amino acid residues or positions in two amino acid sequences represented when the two amino acid sequences are aligned with or without introduction of a gap. The identity between the two amino acid sequences can be determined using a mathamaticl algorithm. Examples of such algorithm include an algorithm described in Karlin and Altshul, Proc. Natl. Acad. Sci. USA 1990, 87: 2264 and an improved algorithm as described in Karlin and Altshul, Proc. Natl. Acad. Sci. USA 1993, 90: 5873-5877. These types of algorithms are incorporated in BLASTN, BLASTX, and the like (Altshul et al., J. Mol. Biol. 1990, 215:403). In order to obtain an amino acid sequence homologous to any one of the polypeptide amino acid sequences shown in SEQ ID NOS: 1 to 15, a BLAST protein search is carried out using the BLAST program (e.g., score = 50; word length = 3). In addition, gapped BLAST (Altshul et al., Nucleic Acid Res. 1997, 25: 3389) can be used to obtain a gapped alignment.

The term "several" as used herein refers to an integer of 10, 9, 8, 7, 6, 5, 4, 3, or 2.

The term "chemically modified derivative" as used herein refers to, but is not limited to, a derivative labeled with a label such as enzyme, fluorophor, dye, or radioisotope, or a derivative having chemical modification such as biotinylation, acetylation, glycosylation, phosphorylation, ubiquitination, or sulfation.

The term "composition or kit for diagnosis and/or detection" as used herein refers to a composition or kit that can be directly or indirectly used for: diagnosing and/or detecting the presence or absence of affection with a disease accompanied with neuropathy such as glaucoma, the degree of affection, the presence or absence of improvement, or the degree of improvement; or screening for a candidate substance useful for prevention, improvement, or treatment of a disease accompanied with neuropathy such as glaucoma.

The term "biological sample" used herein as a subject of detection or diagnosis refers to a sample that contains, or suspected of containing, a target polypeptide that appears along with the development of a disease accompanied with neuropathy such as glaucoma, taken from a living body (e.g., cells, tissue, or body fluid (e.g., blood, lymphatic fluid, or urine))

The term "specifically binding to" as used herein means that an antibody or an antigen-binding fragment thereof forms an antigen-antibody complex with only a target polypeptide (that is, a glaucoma marker in the present invention), a mutant thereof, or a fragment thereof, but does not substantially form such complexes with other peptidic or polypeptidic substances. As used herein, the term "substantially" means that non-specific formation of such complexes may take place, but to a minor extent.

### ADVANTAGE OF THE INVENTION

The markers for a disease accompanied with neuropathy such as glaucoma as defined in the present invention are found in a biological sample such as blood of a patient with glaucoma, but are almost not or are not found in the same of a patient with a different ocular disease such as cataract or age-related macular degeneration. The simple use of the presence or amount of such markers as an indicator provides a significant advantage that glaucoma and a disease accompanied with neuropathy can be easily detected using blood, for example.

This description includes all or part of the contents as disclosed in the description and/or drawings of Japanese Patent Application No. 2008-091522, to which the present application claims a priority.

### BEST MODES FOR CARRYING OUT THE INVENTION

The present invention will be further described specifically as follows.

### <Markers for a disease accompanied with neuropathy>

According to the present invention, markers for diagnosis and/or detection of a disease accompanied with (or associated with) neuropathy using the composition or kit for diagnosis or detection of a disease accompanied with neuropathy such as glaucoma are polypeptides comprising any of amino acid sequences shown in SEQ ID NOS: 1 to 15, mutants thereof, or fragments thereof.

The polypeptides comprising the amino acid sequences shown in SEQ ID NO: 1 to 15 of the present invention are listed in Table 1 below with their protein numbers (Swiss-Prot accession names and numbers.) and their properties. These polypeptides were specifically detected in plasma from patients with glaucoma, whereas they were not detected in plasmas from patients with cataract or age-related macular degeneration, or they were detected at significantly lower levels in plasmas from patients with cataract or age-related macular degeneration than in plasmas from patients with glaucoma. In addition, the amino acid sequences of these polypeptides as shown in the attached SEQUENCE LISTING are available by accessing the Swiss-Prot data bank or the like.

**[Table 1]**

| SEQ ID NO: | Gene name | Protein No. | Properties |
|---|---|---|---|
| 1 | TUBA1A | Q71U36 | Tubulin alpha-1A chain |
| 2 | SAPS1 | Q9UPN7 | SAPS domain family member 1 |
| 3 | LAP1 | Q14847 | LIM and SH3 domain protein 1 |
| 4 | SNAP23 | 000161 | Synaptosomal-associated protein 23 |
| 5 | LTBP1 | Q14766 | Latent-transforming growth factor beta-binding protein, isoform 1L |
| 6 | DBN1 | Q16643 | Drebrin |
| 7 | SRC | P12931 | Proto-oncogene tyrosine-protein kinase Src |
| 8 | TMSB10 | P63313 | Thymosin beta-10 |
| 9 | ZNF185 | 015231 | Zinc finger protein 185 |
| 10 | DNM1L | 000429 | Dynamin-1-like protein |
| 11 | PPP1R12A | 014974 | Protein phosphatase 1 regulatory subunit 12A |
| 12 | PECAM1 | P16284 | Platelet endothelial cell adhesion molecule |
| 13 | TAGLN2 | P37802 | Transgelin-2 |
| 14 | AP2S1 | P53680 | AP-2 complex subunit sigma-1 |
| 15 | XP07 | Q9UIA9 | Exportin-7 |

In the present invention, all of the above target polypeptides for detection of a disease accompanied with neuropathy are characterized in that the polypeptides can be detected only in plasmas of glaucoma patients, or that the levels of the polypeptides in glaucoma patients are significantly or remarkably higher than those in cataract or age-related macular degeneration patients. As used herein, the term "significantly" refers to the presence of a statistically significant difference, wherein the significance level (p) is less than 0.05.

Therefore, when any one of, preferably two or more of, glaucoma marker polypeptide(s) is/are detected in a biological sample of a subject, the occurrence of glaucoma and neuropathy can be determined.

The polypeptides used in the present invention can be prepared by a chemical synthesis method (e.g., peptide synthesis) or a DNA recombination technique, which are conventionally used in the art. The DNA recombination techniques are preferably used in terms of the ease of procedures or purification.

First, polynucleotide sequences encoding partial sequences of the polypeptides of the present invention are chemically synthesized using an automatic DNA synthesizer. The phosphoramidite method is generally employed for such synthesis, which enables the automatic synthesis of a single-stranded DNA with a length of no more than approximately 100 nucleotides. The automatic DNA synthesizer is commercially available from, for example, Polygen or ABI.

With the use of the thus obtained polynucleotides as probes or primers, a cDNA clone of interest is obtained by known cDNA cloning; that is, by constructing a cDNA library via an RT-PCR method from poly A(+)RNA that is obtained by treating total RNA (which is extracted from a tissue of a living body, such as ocular tissue, in which the above target gene is expressed) with an oligo dT cellulose column and then performing screening of the library, such as hybridization screening, expression screening, or antibody screening. If necessary, such cDNA clone can be further amplified by the PCR method. By such procedures, cDNA corresponding to a gene of interest can be obtained.

Probes or primers are selected from sequences of 15 to 100 continuous nucleotides based on the polypeptide sequences shown in SEQ ID NOS: 1-15 and then can be synthesized as described above. Also, cDNA cloning techniques are described in Sambrook, J. and Russel, D., Molecular Cloning, A LABORATORY MANUAL, Cold Spring Harbor Laboratory Press, issued January 15, 2001, Vol. 1, 7.42-7.45 and Vol. 2, 8.9-8.17 and Ausubel et al., Current Protocols in Molecular Biology, 1994, John Wiley & Sons, for example.

Next, the thus obtained cDNA clones are each incorporated into an expression vector and then prokaryotic or eukaryotic host cells transformed or transfected with the vector are cultured, so that a polypeptide of interest can be obtained from the cells or culture supernatants. In this case, a nucleotide sequence encoding a secretory signal sequence may be flanked at the 5' end of a DNA encoding a mature polypeptide of interest, so that the mature polypeptide can be secreted extracellularly.

Vectors and expression systems are available from Novagen, Takara Shuzo, Daiichi Pure Chemicals, Qiagen, Stratagene, Promega, Roche Diagnositics, Invitrogen, Genetics Institute, and Amersham Bioscience, for example. As host cells, prokaryotic cells such as bacteria (e.g., *Escherichia coli* and *Bacillus subtilis*)*,* yeast (e.g., *Saccharomyces cerevisiae*)*,* insect cells (e.g., Sf cell), mammalian cells (e.g., COS, CHO, BHK, and NIH3T3), and the like can be used. Vectors may contain, in addition to DNA encoding the polypeptide, regulatory elements such as a promoter (e.g., lac promoter, trp promoter, P_{L} promoter, P_{R} promoter, SV40 viral promoter, 3-phosphoglycerate kinase promoter, or glycolytic enzyme promoter), an enhancer, a polyadenylation signal, a ribosomal binding site, a replication origin, a terminator, a selection marker (e.g., a drug resistance gene such as ampicillin resistance gene or tetracycline resistance gene; or a complementary auxotrophic markers such as LEU2 or URA3), and the like.

Also, to facilitate purification of a polypeptide, an expression product can also be generated in the form of a fusion polypeptide wherein a peptidic label is bound to the C-terminus or the N-terminus of the polypeptide. Examples of a typical peptidic label include, but are not limited to, a histidine repeat (His tag) comprising 6 to 10 His residues, FLAG, a myc peptide, and a GFP polypeptide.

When the polypeptides according to the present invention are produced without adding any peptidic label, examples of purification methods include ion exchange chromatography. In addition, a combination of techniques including gel filtration chromatography or hydrophobic chromatography, isoelectric point chromatography, high performance liquid chromatography (HPLC), electrophoresis, ammonium sulfate fractionation, salting-out, ultrafiltration, and dialysis may be used. Furthermore, when a peptidic label such as a histidine repeat, FLAG, myc, or GFP is bound to the polypeptide, the purification is carried out using an affinity chromatography appropriate for each peptidic label that is generally used. In this case, an expression vector that makes isolation and purification easy is preferably constructed. In particular, the expression vector is constructed such that a target polypeptide is expressed in the form of a fusion with peptidic label, and the polypeptide is prepared genetic engineeringly using the vector. By doing so, the isolation and purification of the polypeptide can be easily performed.

Purification of nucreic acids can be carried out by purification methods using agalose gel electrophoresis, DNA-binding resin column, and the like. Alternatively, because there are commercially available automated nucleic acid purification systems and nucleic acid purification kits, etc. Purification of nucleic acids may be carried out using such commercially available tools.

As defined above, mutants of the above polypeptides according to the present invention refer to mutants comprising a deletion(s), substitution(s), addition(s), or insertion(s) of one or more, preferably one or several, amino acids in the amino acid sequences shown in SEQ ID NOS: 1-15 or partial sequences thereof; or mutants comprising amino acid sequences showing about 80% or more, about 85% or more, preferably about 90% or more, more preferably about 95% or more, about 97% or more, about 98% or more, or about 99% or more identity with the amino acid sequences or partial sequences thereof. Examples of such mutants include: homologs from mammalian species different from humans; and naturally occurring mutants such as mutants based on polymorphic mutation among mammals of the same species (e.g., race), splice mutants, and natural mutants.

Also, fragments of the above polypeptides of the present invention comprise at least 7, at least 8, at least 10, or at least 15, preferably at least 20, or at least 25, more preferably at least 30, at least 40, at least 50, at least 100, at least 150, or at least 200, or all continuous amino acid residues in the amino acid sequences of the polypeptides, and retain one or more epitopes. Such fragments are capable of immunospecifically binding to antibodies or fragments thereof of the present invention. When the above polypeptides are present in blood, for example, it is assumed that the polypeptides are present as a result of cleavage and fragmentation by an enzyme existing therein such as protease or peptidase.

### <A composition or kit for diagnosis or detection of glaucoma>

According to the present invention, the following is provided: a composition for diagnosis and/or detection of a disease accompanied with neuropathy such as glaucoma, which comprises one or more, preferably 3 or more, more preferably 5 or more, further preferably 10 or more, and most preferably 15 different antibody probes selected from among antibodies, antigen-binding fragments, or chemically modified derivatives thereof capable of specifically binding to polypeptides comprising any of amino acid sequences shown in SEQ ID NOS: 1 to 15, mutants thereof, or fragments thereof.

As used herein, the term "composition" refers not only to a simple mixture of a plurality of antibody probes but also to a combination of the same.

An antibody that recognizes a polypeptide which is a glaucoma marker is capable of specifically binding to the polypeptide via an antigen binding site of the antibody. Such antibody usable in the present invention can be prepared by conventional techniques using polypeptides having the amino acid sequences of SEQ ID NOS: 1-15, mutants thereof, or fragments thereof or using a fusion polypeptide(s) thereof as one or more immunogens. Examples of these polypeptides, mutants thereof, or fragments thereof, or fusion polypeptides include epitopes that induce antibody formation. These epitopes may be linear epitopes or epitopes with higher order structures (discontinuous epitopes). In general, an epitope capable of binding to an antibody is thought to exist on the hydrophilic surface of a polypeptide structure.

Examples of antibodies that can be used in the present invention include antibodies of any types, classes, and subclasses. Examples of such antibodies include IgG, IgE, IgM, IgD, IgA, IgY, IgG1, IgG2, IgG3, IgG4, IgA1, and IgA2.

Moreover, antibodies in all forms are induced by the polypeptides according to the present invention. When the whole or part of the polypeptide or an epitope has been isolated, both polyclonal antibody and monoclonal antibody can be prepared using conventional techniques. An example of such method is as described in Monoclonal Antibodies, Hybridomas: A New Dimension in Biological Analyses, supervised by Kennet et al., Ple num Press, New York, 1980, for example.

A polyclonal antibody can be prepared by immunizing animals such as birds (e.g., chicken) and mammals (e.g., rabbit, goat, horse, sheep, and mouse) with the polypeptide according to the present invention. The antibody of interest can be purified from the blood of immunized animals through an appropriate combination of techniques such as ammonium sulfate fractionation, ion exchange chromatography, and affinity chromatography.

A monoclonal antibody can be obtained by a technique that comprises producing a hybridoma cell line that produces a monoclonal antibody specific to each polypeptide in mice by conventional techniques. One method for producing such hybridoma cell line comprises immunizing animals with the polypeptide according to the present invention, collecting spleen cells from immunized animals, fusing the spleen cells to a myeloma cell line so as to generate hybridoma cells, and then identifying the hybridoma cell line that produces a monoclonal antibody binding to the polypeptide. The monoclonal antibody can be collected by conventional techniques.

Preparation of monoclonal and polyclonal antibodies is described in detail as follows.

### A. Preparation of monoclonal antibody

### (1) Immunization and collection of antibody-producing cell

An immunogen obtained as described above is administered to a mammal such as a rat, a mouse (e.g., the inbred mouse strain Balb/c), or a rabbit. The dose of the immunogen can be appropriately determined depending on, for example, the type of an animal to be immunized or the route of administration, and it is about 50 µg to 200 µg per animal. Immunization is primarily performed by injecting an immunogen subcutaneously or intraperitoneally. Also, the intervals of immunization are not particularly limited. After the primary immunization, boost immunization is carried out 2 to 10 times, and preferably 3 or 4 times, at intervals of several days to several weeks, and preferably at intervals of 1 to 4 weeks. After the primary immunization, the antibody titer of the blood serum of the immunized animal is repeatedly measured by, for example, ELISA (Enzyme-Linked Immuno Sorbent Assay). When the antibody titer reaches a plateau, the immunogen is injected intravenously or intraperitoneally to complete the final immunization. Antibody-producing cells are collected 2 to 5 days and preferably 3 days after the final immunization. Examples of antibody-producing cells include spleen cells, lymph node cells, and peripheral blood cells, and preferably spleen cells or regional lymph node cells.

### (2) Cell fusion

Hybridoma cell lines that produce monoclonal antibodies specific to each protein can be produced and then identified by conventional techniques. A method for producing such hybridoma cell lines comprises immunizing an animal with the polypeptide of the invention, removing spleen cells from the immunized animal, fusing the spleen cells to a myeloma cell line, producing hybridoma cells therefrom, and then identifying a hybridoma cell line that produces a monoclonal antibody binding to the polypeptide of interest. Myeloma cell lines to be fused to antibody-producing cells, which can be used herein, are commercially available established cell lines of animals such as mice. Preferably, cell lines to be used herein have drug selectivity so that they cannot survive in a HAT selective medium (containing hypoxanthine, aminopterin, and thymidine) in an unfused state, but they can survive only in a state fused to antibody-producing cells. Such established cell lines are preferably derived from an animal of the same species with the immunized animal. A specific example of the myeloma cell line is a P3X63-Ag.8 strain (ATCC TIB9), which is a BALB/c mouse-derived hypoxanthine·guanine·phosphoribosyl·transferase (HGPRT) deficient cell line.

Subsequently, the myeloma cell lines are fused to the antibody-producing cells. Cell fusion is carried out in a serum-free medium for animal cell culture, such as DMEM or RPMI-1640 medium, by mixing the antibody-producing cells with the myeloma cell lines at about 1:1 to 20:1 in the presence of a cell fusion accelerator. As the cell fusion accelerator, polyethylene glycol or the like having an average molecular weight ranging from 1,500 to 4,000 daltons can be used at a concentration ranging from about 10% to 80%, for example. Optionally, an auxiliary agent, such as dimethyl sulfoxide, can be used in combination to enhance the fusion efficiency. Further, the antibody-producing cells can be fused to the myeloma cell lines using a commercially available cell fusion apparatus utilizing electric stimuli (e.g., electroporation).

### (3) Selection and cloning of hybridoma

The hybridomas of interest are selected from the fused cells. To this end, the cell suspension is adequately diluted with, for example, a fetal bovine serum-containing RPMI-1640 medium, then the suspension is aliquoted into each well of a microtiter plate at about two million cells/well, a selection medium is added to each well, and then culture is carried out while appropriately exchanging the selection medium with the same fresh medium. The culture temperature ranges from 20°C to 40°C and is preferably about 37°C. When the myeloma cell is an HGPRT-deficient cell line or thymidine kinase-deficient cell line, only a hybridoma of a cell having an ability to produce an antibody and a myeloma cell line can selectively be cultured and grown in the selection medium containing hypoxanthine, aminopterin, and thymidine (i.e., the HAT medium). As a result, cells that start to grow on about day 14 after the initiation of culture in the selection medium can be obtained as hybridoma cells.

Subsequently, whether or not the culture supernatant of the grown hybridomas contains the antibody of interest is screened for. Screening of hybridomas can be carried out in accordance with conventional techniques, without particular limitation. For example, the culture supernatant in a well containing the grown hybridomas is partially sampled and then subjected to enzyme immuno assay (EIA) or ELISA or radio immuno assay (RIA). The fused cells are cloned using the limiting dilution method or the like, and monoclonal antibody-producing cells, i.e. hybridomas, are established in the end. The hybridoma is stable during culture in a basic medium, such as RPMI-1640 or DMEM, and the hybridoma can produce and secrete a monoclonal antibody that reacts specifically with a polypeptidic marker for glaucoma of the present invention.

### (4) Recovery of antibody

Monoclonal antibodies can be recovered by conventional techniques. Specifically, a monoclonal antibody can be collected from the established hybridoma by a conventional cell culture technique, ascites development, or the like. According to the cell culture technique, hybridomas are cultured in an animal cell culture medium, such as 10% fetal bovine serum-containing RPMI-1640 medium, MEM medium, or a serum-free medium, under common culture conditions (e.g., 37°C, 5% CO₂ concentration) for 2 to 10 days, and the antibody is obtained from the culture supernatant. In the case of ascites development, about 10 millions of hybridoma cells are administered intraperitoneally to an animal of the same species as the mammal from which the myeloma cells are derived, so as to allow the hybridoma cells to grow in large quantity. After one to two weeks, the ascites or blood serum is taken from the animal.

Where antibody purification is required in the above-described method for collecting the antibody, known techniques, such as salting out with ammonium sulfate, ion-exchange chromatography, affinity chromatography, and gel filtration chromatography, may be appropriately selected or combined to obtain the purified monoclonal antibody of the present invention.

### B. Preparation of polyclonal antibody

When polyclonal antibodies are prepared, an animal is immunized in the same manner as described above, the antibody titer is measured on days 6 to 60 after the final immunization by enzyme immuno assay (EIA or ELISA) or radio immuno assay (RIA), and blood is taken on the day when the maximal antibody titer is measured, in order to obtain antiserum. Thereafter, the reactivity of the polyclonal antibodies in the antiserum is measureed by ELISA or the like.

Also, in the present invention, an antigen-binding fragment of the above antibodies can also be used. Examples of antigen-binding fragments that can be produced by conventional techniques include, but are not limited to, Fab and F(ab')₂, Fv, scFv, and dsFv. Examples thereof also include antibody fragments and derivatives thereof that can be produced by genetic engineering techniques. Examples of such antibodies include synthetic antibodies, recombinant antibodies, multi-specific antibodies (including bispecific antibodies), and single chain antibodies.

The antibodies of the present invention can be used *in vitro* and *in vivo.* In the present invention, the antibodies can be used in assays for detection of the presence of polypeptides or (poly)peptide fragments thereof. A monoclonal antibody is preferably used to enable specific detection in the assay. Even in the case of a polyclonal antibody, a specific antibody can be obtained by a so-called absorption method that comprises binding an antibody to an affinity column to which a purified polypeptide is bound.

Therefore, the composition of the present invention can contain at least one, preferably a plural number of types of (e.g., two or three types or more), and more preferably all types of antibodies or antigen-binding fragments thereof capable of specifically binding to the polypeptides comprising amino acid sequences of SEQ ID NOS: 1-15, mutants thereof, or fragments thereof.

A label, such as a fluorophore, an enzyme, or a radioisotope may be bound to an antibody or an antigen-binding fragment thereof to be used in the present invention, if necessary.

Examples of a fluorophore include fluorescein and a derivative thereof, rhodamine and a derivative thereof, dansyl chloride and a derivative thereof, and umbelliferone.

Examples of an enzyme include horseradish peroxidase and alkaline phosphatase.

Examples of a radioisotope include iodines (¹³¹I, ¹²⁵I, ¹²³ I, and ¹²¹I) phosphorus ³²P , sulfur (³⁵S) and metals (e.g., ⁶⁸Ga , ⁶⁷Ga, ⁶⁸Ge, ⁵⁴Mn, ⁹⁹Mo, ⁹⁹Tc , and ¹³³Xe).

Examples of other labels include luminescence substances such as luminol and bioluminescence substances such as luciferase and luciferin.

Also, if necessary, an avidin-biotin system or a streptavidin-biotin system can also be used herein. In this case, for example, biotin can be bound to the antibody or an antigen-binding fragment thereof of the present invention.

The present invention further provides a kit for diagnosis and/or detection of neuropathy, preferably ocular neuropathy, more preferably glaucoma, which comprises one or more antibody probes selected from antibodies or antigen-binding fragments thereof, or chemically modified derivatives thereof capable of specifically binding to at least one polypeptide comprising any of the amino acid sequences shown in SEQ ID NOS: 1 to 15, a mutant thereof, or a fragment thereof.

In this context, the present invention further provides a use of one or more antibody probes selected from among antibodies or antigen-binding fragments thereof, or chemically modified derivatives thereof capable of specifically binding to at least one polypeptide comprising any of the amino acid sequences shown in SEQ ID NOS: 1 to 15, a mutant thereof, or a fragment thereof for production of the above-described kit.

The kit comprises, for examples, individual containers (e.g., vials) in which the above-described antibody probes for detection of glaucoma markers are packaged individually or, appropriately, in admixture. Preferably, antibody probes may be packaged in the lyophilized state in containers.

Alternatively, the kit of the present invention may comprise a solid-phase support comprising a multi-well plate, an array, a microtiter plate, a test piece, spherical carriers such as latex beads or magnetic beads, or the like, to which antibodies or fragments thereof capable of specifically binding to the aforementioned polypeptides have been attached or (covalently or non-covalently) bonded.

Further, the kit of the present invention may contain a buffer, a secondary antibody, instructions, and the like, which are used in the assay method of the present invention.

Instead of the above-described antibody probes, nucleic acid probes can be used in the method, composition, and kit of the present invention. The nucleic acid probes are DNAs, which code for polypeptides comprising any of amino acid sequences shown in SEQ ID NOS: 1-15 as described above, mutants thereof, or fragments thereof. The nucleic acid probes can be produced by the above-described method for producing the corresponding polypeptides by gene recombination technology. Mutants or fragments thereof can be produced by, for example, PCR using appropriate primers and parent polypeptides as templates. The nucleic acid probes can generally have a size of approximately 15-100 nucleotides or more and preferably approximately 20-80 nucleotides. In addition, with the use of nucleic acid probes, DNA-DNA hybridization, DNA-RNA hybridization, RNA-RNA hybridization, or the like is performed under stringent conditions such that target markers are detected. Regarding hybridization conditions, for example, conditions described in Sambrook, J. and Russel, D., Molecular Cloning, A LABORATORY MANUAL, Cold Spring Harbor Laboratory Press, published on January 15, 2001, Vol. 1, 7.42-7.45, Vol. 2, 8.9-8.17, or Ausubel et al., Current Protocols in Molecular Biology, 1994, John Wiley & Sons, etc. can be employed.

### <Detection of a disease accompanied with neuropathy>

According to the present invention, a disease accompanied with neuropathy can be detected by a method that comprises determining *in vitro* the presence or amount of one or more of the polypeptides comprising the amino acid sequences shown in SEQ ID NOS: 1-15, mutants thereof, or fragments thereof in a biological sample from a subject using substances capable of binding to the above-described markers. In a possible diagnosis conducted by the method of the present invention, where the glaucoma marker(s) is/are detected or the gene expression levels are determined to be significantly higher than control levels, a subject is determined to be in the advanced stage of neuropathy, thus to suffer from ocular neuropathy, partcularly glaucoma.

In the method of the present invention, the detection of markers for a disease accompanied with neuropathy may be performed using a single marker, but is preferably performed using a plurality of (e.g., from 2 or more, 3 or more, 4 or more, or 5 or more, to 22) markers. This is intended to avoid unpredictable detection of a non-specific complex, in other words, misdiagnosis.

The composition or kit of the present invention is useful for diagnosis, determination, or detection of a disease accompanied with neuropathy, i.e., for diagnosis of the presence or absence of the disease or the degree of the disease. In diagnosis of a disease accompanied with neuropathy, comparison is made with negative controls such as normal cells, normal tissues, or normal body fluids, and then the presence or amount of the above-described glaucoma markers in a biological sample from a subject is detected. When a difference in the presence or amount is found to be significant, the subject is suspected of advanced neuropathy or suffering from glaucoma.

Examples of test samples used in the present invention include body fluids such as blood, serum, blood plasma, and urine.

Examples of the above-described substances capable of binding to glaucoma markers include not only the above-described antibodies or antigen-binding fragments thereof but also, for example, aptamers, Affibody^{™} (Affibody), receptors of the glaucoma markers, substances inhibiting the specific action of the glaucoma markers, and substances activating the specific action of the glaucoma markers, preferably antibodies or antigen-binding fragments thereof or chemically modified derivatives thereof.

In an embodiment of the present invention, the measurement can comprise the steps of: bringing an antibody or fragment thereof, which may be optionally labeled with a conventional enzyme or fluorophore, into contact with a tissue section or a homogenized tissue or a body fluid; and qualitatively or quantitatively measuring an antigen-antibody complex. The detection is carried out by, for example, a method for measuring the presence and the level of a target polypeptide by immunoelectron microscopy, or a method for measuring the presence or the levels of target polypeptides by a conventional method such as an enzyme antibody method (e.g., ELISA), a fluorescent antibody technique, a radioimmunoassay, a homogeneous method, a heterogeneous method, a solid phase method, or a sandwich method. Where the target polypeptide is found to be present in a body fluid or an glaucoma tissue or cells, preferably blood, obtained from a subject, or the level of the target polypeptide is found to be significantly increased or higher than the negative control level, the subject is determined to have glaucoma. As used herein, the term "significantly" refers to the presence of a statistically significant difference (p<0.05).

An example of a measurement method as an alternative for an immunological method is a method using mass spectrometry. This method can be performed specifically by procedures described in the Examples. Specifically, a biological sample such as serum or blood plasma is filtered using a filter to remove contaminants, diluted with a buffer (e.g., pH, about 8), and then adjusted to have a concentration ranging from about 10 mg/ml to about 15 mg/ml. Subsequently, the resultant is filtered through a hollow fiber filter (Reference Example (1) below) or a centrifugal flat membrane filter, which is capable of removing proteins with a molecular weight of 50,000 or more, so as to perform molecular weight fractionation. The fractions are treated with protease (e.g., trypsin) for peptidization and then the resultants are subjected to a mass spectrometer (the type using matrix-assisted laser desorption ionization or electrospray ionization). Differences between the amount of a polypeptide existing in a sample of a patient with glaucoma and the same of a healthy subject or a patient with a different ocular disease can be measured based on the mass-to-charge ratio (m/z) and intensity at a specific peak from the polypeptide of interest.

### EXAMPLES

The present invention will be described in more detail with reference to the examples set forth below; however, the technical scope of the present invention is not limited to the examples.

### <Reference Example>

### (1) Preparation of hollow fiber filter

A hundred polysulfone hollow fibers having a pore size (molecular weight cut off) of approximately 50,000 on the membrane surface were packed into a bundle. The both ends of the bundle were fixed to a glass tube using an epoxy-based potting agent so as not to occlude the hollow parts of the hollow fibers, so that a mini module is prepared. The mini module (module A) was used for removal of high-molecular-weight proteins in serum or blood plasma, having a diameter of about 7 mm and a length of about 17 cm. Similarly, a mini module (module B) to be used for concentrating low-molecular-weight proteins was prepared using a membrane with a pore size (molecular weight cut off) of approximately 3,000. The mini modules have an inlet that is connected to hollow fiber lumen on one end and an outlet on the other end. The inlets and outlets of hollow fibers constitute flow passages of a closed circulatory system formed via a silicon tube. Through the flow passages, a liquid is driven by a Peristar pump to circulate. Also, a glass tube of the hollow fiber mantle is provided with a port for discharging a liquid leaking from the hollow fibers, so that one module set is constituted. The modules were connected to a position in the middle of such flow passage via a "T"-shaped connector, i.e., three modules A and one module B were connected in tandem, thereby forming one hollow fiber filter. The hollow fiber filter was washed with distilled water, and then filled with an aqueous 25 mM ammonium bicarbonate solution (pH 8.2). A fraction raw material (i.e., serum or blood plasma) was injected from the flow passage inlet of the hollow fiber filter and then discharged from the passage outlet after fractionation and concentration. Serum or blood plasma injected to the hollow fiber filter was applied to a molecular sieve with a molecular weight cut off of approximately 50,000 for every module A. Thus, components with molecular weights lower than that of 50,000 are concentrated using the module B and then prepared.

### <Example 1>

### (1) Idendification of plasma proteins in normal tension-glaucoma patients, cataract patients, and age-related macular degeneration patients

Heparinized plasmas were obtained for measurement, from 10 patients with age-related macular degeneration (aged 82 on average), 10 patients with cataract and 10 patients with normal tension-glaucoma of similar age. The blood plasmas were centrifuged to remove contaminants, and the resulting plasmas were further diluted with 25 mM ammonium bicarbonate solution (pH 8.2) to a concentration of 12.5 mg/ml, followed by carrying out a molecular weight fractionation using the hollow fiber filter as described in Reference Example (1). Each fractionated blood plasma sample (total amount of 1.8 ml, comprising 250 µg (max) of proteins) was separated into 3 fractions by reversed-phase chromatography with AKTA explorer 10s (GE Healthcare Biosciences). The fractions were each lyophilized and then redissolved in 8 M urea solution. The samples were treated with DTT·iodoacetamide and then diluted 10-fold, followed by overnight digestion at 37°C with trypsin (at a ratio 1:50 of trypsin to proteins) for peptidization. After removal of urea using a desalting column, peptides in each fraction were further fractionated into 8 fractions using an ion-exchange column. Each resulting fraction was further fractionated using a reverse-phase column, and the eluted peptides were subjected to mass spectrometry with an online-connected mass spectrometer (LCQ Deca XP plus; Thermo Fisher Scientific K.K.).

### (2) Comparison of the expressed plasma proteins among normal tension-glaucoma patients, cataract patients, and age-related macular degeneration patients

Data determined in (1) above were analyzed using the protein identification softwares Bioworks (Thermo Fisher Scientific K.K.) and Phenyx (GENE BIO) for comprehensive protein identification. From among identified proteins, proteins identified by the two different types of software were listed and designated as proteins detected from plasma samples of patients with each disease. This was carried out to exclude false-positive proteins contained in analysis results of either one of the softwares by combining the two different softwares having different algorithms. However, unlike Bioworks, Phenyx carries out searching in consideration of isoform-specific amino acid sequences obtained by alternative splicing of an identidal protein and changes in mass after post-translation modification. Hence, the software Phenyx might identify peptides that cannot be identified by Bioworks. Under the above conditions, proteins identified only by Phenyx were also listed.

Among proteins listed for each disease, proteins detected from normal tension-glaucoma patients but never from cataract patients or age-related macular degeneration patients were found as plasma marker proteins. These proteins correspond to polypeptides comprising any of amino acid sequences shown in SEQ ID NOS: 1 to 15 in Table 1 (above) and the SEQUENCE LISTING. Accordingly, it was revealed that the proteins are useful as glaucoma markers for detection of glaucoma or for diagnostic determination of the progression of glaucoma during treatment.

### INDUSTRIAL APPLICABILITY

The present invention provides the compositions or kits with good specificity and sensitivity for diagnosis of a disease accompanied with neuropathy such as glaucoma, and it is particularly useful in the pharmaceutical and medical industries.

All publications, patents, and patent applications cited herein are incorporated herein by reference in their entirety.

## Claims

1. A method for determining neuropathy, comprising quantitatively or qualitatively measuring and/or detecting one or more of polypeptides comprising the amino acid sequences shown in SEQ ID NOS: 1 to 15, mutants thereof, or fragments thereof, in a biological sample from a subj ect.

2. The method according to claim 1, wherein the neuropathy is ocular tissue neuropathy.

3. The method according to claim 2, wherein the ocular tissue neuropathy is glaucoma.

4. The method according to any one of claims 1 to 3, wherein the measurement and/or detection of the polypeptide, a mutant thereof, or a fragment thereof is carried out by mass spectrometry.

5. The method according to claim 4, wherein the measurement and/or detection is carried out using a substance capable of binding to the polypeptide, a mutant thereof, or a fragment thereof.

6. The method according to claim 5, wherein the substance capable of binding is an antibody or an antigen-binding fragment thereof.

7. The method according to claim 6, wherein the antibody labeled with any of an enzyme, a fluorophor, a dye, a radioisotope, or biotin is used.

8. The method according to claim 6 or 7, wherein the antibody or an antigen-binding fragment thereof is a monoclonal antibody or a polyclonal antibody, or an antigen-binding fragment thereof.

9. The method according to any one of claims 1 to 8, wherein the biological sample is blood, plasma, or serum.

10. A composition for diagnosis and/or detection of neuropathy, which comprises one or more antibody probes selected from antibodies, antigen-binding fragments, or chemically modified derivatives thereof capable of specifically binding to at least one of polypeptides comprising any of amino acid sequences shown in SEQ ID NOS: 1 to 15, mutants thereof, or fragments thereof.

11. A kit for diagnosis and/or detection of neuropathy, which comprises one or more antibody probes selected from antibodies, antigen-binding fragments, or chemically modified derivatives thereof capable of specifically binding to at least one of polypeptides comprising any of amino acid sequences shown in SEQ ID NOS: 1 to 15, mutants thereof, or fragments thereof.

12. The composition or kit according to claim 10 or 11, wherein the neuropathy is ocular tissue neuropathy.

13. The composition or kit according to claim 12, wherein the ocular tissue neuropathy is glaucoma.

14. Use of one or more antibody probes selected from antibodies, antigen-binding fragments, or chemically modified derivatives thereof capable of specifically binding to at least one of polypeptides comprising any of amino acid sequences shown in SEQ ID NOS: 1 to 15, mutants thereof, or fragments thereof, in production of the kit according to any one of claims 11-13.
